# EUROPEAN PATENT APPLICATION

(11) **EP 4 818 649 A1**
(43) Date of publication of application: **07.10.2026**
(21) Application number: 24898168.0
(22) Date of filing: 28.11.2024
(51) Int. Cl.: G01D 11/24, G01D 11/26, H01R 12/79, H01R 12/70, H01R 12/71

(54) **ANALYTE MONITORING DEVICE**

(30) Priority: 28.11.2023 KR 20230168332; 01.12.2023 KR 20230172661
(71) Applicant: SD Biosensor, Inc., Suwon-si, Gyeonggi-do 16690 (KR)
(72) Inventor: LEE, Hyo Keun, Suwon-si, Gyeonggi-do 16690 (KR); PARK, Hyo Lim, Suwon-si, Gyeonggi-do 16684 (KR); LEE, Jun Hwang, Yongin-si, Gyeonggi-do 16999 (KR); KIM, Dong Hun, Suwon-si, Gyeonggi-do 16700 (KR); JEON, Sang Yeun, Yongin-si, Gyeonggi-do 17091 (KR); HONG, Soon Min, Hwaseong-si, Gyeonggi-do 18477 (KR); LEE, Sang Min, Suwon-si, Gyeonggi-do 18477 (KR); KIM, Iek Lok, Suwon-si, Gyeonggi-do 16690 (KR); KIM, Jong Gwang, Hwaseong-si, Gyeonggi-do 18456 (KR); YUN, Jin Seok, Hwaseong-si, Gyeonggi-do 18472 (KR); LEE, Jeong Hwan, Hwaseong-si, Gyeonggi-do 18466 (KR)
(74) Representative: Berggren Oy
(86) International application number: PCT/KR2024/019187
(87) International publication number: WO 2025/116574

(57) **Abstract**

An analyte monitoring device of the present invention includes a housing forming an inner space, a sensor portion including a connecting portion located in the inner space and a sensing portion extending from the connecting portion and exposed to the outside of the housing, and a circuit portion located in the inner space and including a contact portion in contact with the connecting portion, wherein the contact portion is formed of a flexible material and is formed to extend in a horizontal direction from a portion adjacent to a body portion of the circuit portion and then bend upward or downward due to contact with the connecting portion.

## Description

### [Technical Field]

The present invention relates to a device that is inserted into the body to monitor an analyte.

### [Background Art]

A recent continuous glucose monitoring (CGM) device is a system in which a fine sensor is inserted into the skin of a diabetic patient to continuously measure blood glucose. The corresponding device consists of a sensor and an electronic device, and the electronic device processes and records an analyte (blood glucose) measured through the sensor and provides real-time blood glucose information to a patient. The CGM device is usually attached to the skin and provides a function of continuously monitoring blood glucose for about 7 to 15 days, and in this way, more precise blood glucose management is possible for the patient.

However, the conventional CGM device has technical limitations. In particular, there is technical difficulty in stably fixing a sensor and accurately connecting the sensor and a circuit in a process of designing a miniaturized device. Such technical problems may degrade the performance and reliability of the device and have a negative influence also on commercialization of the device and convenience of the patient.

Therefore, there is need for development of a technology that can improve connectivity between a sensor and a circuit and allows the sensor to be stably fixed in a miniaturized device for monitoring an analyte (blood glucose).

### [Disclosure]

### [Technical Problem]

The present invention is directed to providing an analyte monitoring device capable of improving connectivity between a sensor and a circuit and allowing the sensor to be stably fixed in a miniaturized device for monitoring an analyte.

The present invention is directed to providing an analyte monitoring device in which a contact maintaining portion and a coupling maintaining portion are added to more firmly fix a sensor and a contact portion, thereby providing a consistent measurement result even during long-term use.

The present invention is directed to providing an analyte monitoring device in which a close contact maintaining portion and a sensor fixing portion are added to more firmly fix a sensor and a contact portion, thereby providing a consistent measurement result even during long-term use.

### [Technical Solution]

An analyte monitoring device of the present invention includes a housing forming an inner space, a sensor portion including a connecting portion located in the inner space and a sensing portion extending from the connecting portion and exposed to the outside of the housing, and a circuit portion located in the inner space and including a contact portion in contact with the connecting portion, wherein the contact portion is formed of a flexible material and is formed to extend in a horizontal direction from a portion adjacent to a body portion of the circuit portion and then bend upward or downward due to contact with the connecting portion.

In one embodiment of the present invention, the body portion may be formed as a rigid circuit board.

In one embodiment of the present invention, the body portion may be formed as a circuit board including a plurality of layers that are stacked, and the contact portion may be formed as a flexible conductive member that extends from at least one of the plurality of layers.

In one embodiment of the present invention, the contact portion may be a flexible circuit board coupled to the body portion.

In one embodiment of the present invention, the contact portion may include a horizontal extending portion that is adjacent to the body portion and extends in the horizontal direction and a bending portion that extends from an end of the horizontal extending portion and bends upward or downward due to the contact with the connecting portion.

In one embodiment of the present invention, the contact portion may further include a contact extending portion that extends from an end of the bending portion and extends parallel to the connecting portion while in contact therewith.

In one embodiment of the present invention, the connecting portion may be formed in the form of a plate that stands upright in a vertical direction.

In one embodiment of the present invention, the connecting portion may include a sensor terminal formed on a surface facing the contact portion, the contact portion may include a contact terminal formed on a lower surface, and the sensor terminal and the contact terminal may come into contact as the contact portion bends upward due to the contact with the connecting portion.

In one embodiment of the present invention, the connecting portion may include a first sensor terminal formed on a first surface and a second sensor terminal formed on a second surface opposite to the first surface, and the contact portion may include a first contact portion that extends from a first end of the body portion toward the first surface and comes into contact with the first sensor terminal and a second contact portion that extends from a second end of the body portion toward the second surface and comes into contact with the second sensor terminal.

In one embodiment of the present invention, the analyte monitoring device may further include a contact maintaining portion that maintains a contact state between the connecting portion and the contact portion.

In one embodiment of the present invention, the contact maintaining portion may include an extending portion that extends in the inner space of the housing and a coupling portion that is coupled to the extending portion so that the connecting portion and the contact portion that are in contact with each other are located between the coupling portion and the extending portion.

In one embodiment of the present invention, the extending portion may include a protruding portion that protrudes from the housing and comes into contact with any one of the connecting portion and the contact portion and a spaced portion that protrudes from the housing and is spaced from the protruding portion, and the coupling portion may be coupled between the protruding portion and the spaced portion, may come into contact with the other of the connecting portion and the contact portion, and may allow the connecting portion and the contact portion to come in close contact with each other.

In one embodiment of the present invention, the extending portion may include a protruding portion that protrudes from the housing and comes into contact with any one of the connecting portion and the contact portion, and the coupling portion may rotate about a rotating shaft to be adjacent to the protruding portion, may come into contact with the other of the connecting portion and the contact portion, and may allow the connecting portion and the contact portion to come in close contact with each other.

In one embodiment of the present invention, the extending portion may include a first protruding portion that protrudes from the housing and comes into contact with a second surface of a first portion of the connecting portion and a second protruding portion that protrudes from the housing and comes into contact with a first surface of a second portion of the connecting portion, and the coupling portion may include a first coupling portion that is coupled from the first surface to the second protruding portion and maintains a contact state between the first sensor terminal and the first contact portion and a second coupling portion that is coupled from the second surface to the first protruding portion and maintains a contact state between the second sensor terminal and the second contact portion.

In one embodiment of the present invention, the analyte monitoring device may further include a coupling maintaining portion that maintains a coupling state between the extending portion and the coupling portion.

An analyte monitoring device of the present invention includes a housing forming an inner space, a sensor portion including a connecting portion located in the inner space and having at least one sensor terminal formed thereon and a sensing portion extending from the connecting portion and exposed to the outside of the housing, a circuit portion located in the inner space and including a signal processing portion, and a sensor holder that includes a first contact portion and a second contact portion in contact with a first surface and a second surface, respectively, of the connecting portion and electrically connects the sensor terminal and the circuit portion.

In one embodiment of the present invention, at least one of the first contact portion and the second contact portion may be a transfer contact portion including a signal transfer portion connected to the sensor terminal and the circuit portion.

In one embodiment of the present invention, the transfer contact portion may be formed of a resin material including an additive that is nonconductive and then turns conductive when light of a specific condition is radiated thereto, the transfer contact portion may include, on one portion of a surface thereof, a light radiation region in which the additive turns conductive due to the light of the specific condition being radiated thereto, and the transfer contact portion may be a plated layer coupled to the light radiation region.

In one embodiment of the present invention, the sensor holder may further include a close contact maintaining portion that maintains a state in which the first contact portion and the second contact portion are in close contact with each other with the connecting portion disposed therebetween.

In one embodiment of the present invention, the sensor holder may form a sensor accommodating space accommodating a portion of the connecting portion that is adjacent to the sensing portion, and a through-hole through which the sensing portion passes may be formed in a portion of the housing that is located at a lower portion of the sensor accommodating space.

### [Advantageous Effects]

The present invention has advantages that it is possible to improve connectivity between a sensor and a circuit and allow the sensor to be stably fixed in a miniaturized device for monitoring an analyte.

The present invention has advantages that, since a contact maintaining portion and a coupling maintaining portion are added, a sensor and a contact portion can be more firmly fixed, and thus a consistent measurement result can be provided even during long-term use.

The present invention has advantages that, since a close contact maintaining portion and a sensor fixing portion are added, a sensor and a contact portion can be more firmly fixed, and thus a consistent measurement result can be provided even during long-term use.

### [Description of Drawings]

FIG. 1 is a perspective view of an analyte monitoring device according to one embodiment of the present invention.
FIG. 2 is a bottom perspective view of the analyte monitoring device according to one embodiment of the present invention.
FIG. 3 is an exploded perspective view of the analyte monitoring device according to one embodiment of the present invention.
FIG. 4 is a perspective view of a circuit portion according to one embodiment of the present invention.
FIG. 5 is a cross-sectional view of the circuit portion according to one embodiment of the present invention.
FIG. 6 is a perspective view of a state before a lower housing and a sensor portion are coupled according to one embodiment of the present invention.
FIG. 7 is a perspective view of a state before the lower housing and the circuit portion are coupled according to one embodiment of the present invention.
FIG. 8 is a perspective view of a state in which the lower housing and the circuit portion are coupled according to one embodiment of the present invention.
FIG. 9 is a cross-sectional view of the state in which the lower housing and the circuit portion are coupled according to one embodiment of the present invention.
FIG. 10 is a perspective view of a state in which the lower housing, the circuit portion, and a contact maintaining portion are formed according to one embodiment of the present invention.
FIG. 11 is a cross-sectional view of the state in which the lower housing, the circuit portion, and the contact maintaining portion are formed according to one embodiment of the present invention.
FIG. 12 is a perspective view of a state in which the lower housing, the circuit portion, the contact maintaining portion, and a coupling maintaining portion are coupled according to one embodiment of the present invention.
FIG. 13 is a cross-sectional view of the state in which the lower housing, the circuit portion, the contact maintaining portion, and the coupling maintaining portion are coupled according to one embodiment of the present invention.
FIG. 14 is a perspective view of a state in which the lower housing and a circuit portion are coupled according to one embodiment of the present invention.
FIG. 15 is a perspective view of a state in which the lower housing, the circuit portion, and the contact maintaining portion are coupled according to one embodiment of the present invention.
FIG. 16 is a cross-sectional view of a state in which the lower housing, the circuit portion, the contact maintaining portion, the coupling maintaining portion, and an upper housing are coupled according to one embodiment of the present invention.
FIG. 17 is a cross-sectional view of a sensor portion including a sensor terminal according to one embodiment of the present invention.
FIG. 18 is a perspective view of a state in which the lower housing and the circuit portion are coupled according to one embodiment of the present invention.
FIG. 19 is a cross-sectional view of the state in which the lower housing and the circuit portion are coupled according to one embodiment of the present invention.
FIG. 20 is a perspective view of a state in which the lower housing, the circuit portion, and the contact maintaining portion are coupled according to one embodiment of the present invention.
FIG. 21 is a perspective view of a lower surface of an upper housing according to one embodiment of the present invention.
FIG. 22 is a cross-sectional view of a state in which the lower housing, the circuit portion, the contact maintaining portion, the coupling maintaining portion, and the upper housing are coupled according to one embodiment of the present invention.
FIG. 23 is a perspective view of an analyte monitoring device according to one embodiment of the present invention.
FIG. 24 is a bottom perspective view of the analyte monitoring device according to one embodiment of the present invention.
FIG. 25 is an exploded perspective view of the analyte monitoring device according to one embodiment of the present invention.
FIG. 26 is a view separately illustrating either surface of a sensor portion according to one embodiment of the present invention.
FIG. 27 is an exploded perspective view of a contact portion according to one embodiment of the present invention.
FIG. 28 is an exploded perspective view of a first contact portion, a second contact portion, and the sensor portion according to one embodiment of the present invention.
FIG. 29 is a perspective view of a state in which the first contact portion, the second contact portion, and the sensor portion are coupled according to one embodiment of the present invention.
FIG. 30 is a partial exploded perspective view of the sensor portion, the contact portion, and a close contact maintaining portion according to one embodiment of the present invention.
FIG. 31 is a perspective view of a state in which the sensor portion, the contact portion, and the close contact maintaining portion are coupled according to one embodiment of the present invention.
FIG. 32 is a partial exploded perspective view of the sensor portion, a sensor holder, and a sensor fixing portion according to one embodiment of the present invention.
FIG. 33 is a cross-sectional view of a state in which the sensor portion, the sensor holder, and the sensor fixing portion are coupled according to one embodiment of the present invention.
FIG. 34 is an exploded perspective view of a state in which the upper housing is open in the analyte monitoring device according to one embodiment of the present invention.
FIG. 35 is an exploded perspective view of the analyte monitoring device according to one embodiment of the present invention.
FIG. 36 is an exploded perspective view of a contact portion according to another embodiment of the present invention.
FIG. 37 is a see-through top view of a contact portion according to still another embodiment of the present invention.
FIG. 38 is an exploded perspective view of a sensor portion and a contact portion according to yet another embodiment of the present invention.
FIG. 39 is a perspective view of a coupling state of the sensor portion and a sensor holder according to yet another embodiment of the present invention.

### [Modes of the Invention]

Hereinafter, the embodiments disclosed herein will be described in detail with reference to the accompanying drawings. Throughout the drawings, the same or similar components will be denoted by the same reference numerals, and redundant description thereof will be omitted. In addition, when detailed description of relevant prior art in explaining the embodiments disclosed in this specification is deemed to potentially obscure the gist of the disclosed embodiments, the detailed description will be omitted.

Terms including ordinals such as "first" and "second" may be used to describe various components, but the components are not limited by the terms. The terms are only used for the purpose of distinguishing one component from another component.

A singular expression includes a plural expression unless the context clearly indicates otherwise.

In this application, operations described may be performed regardless of a listed order, except for a case where they must be performed in the listed order due to a special causal relationship.

In this application, the terms "include" or "have" should be understood as specifying the presence of stated features, integers, steps, operations, components, parts, or combinations thereof, instead of precluding the presence or addition of one or more other features, integers, steps, operations, components, parts, or combinations thereof.

An analyte monitoring device of the present invention may be a device that is attached to the body of a user to measure and monitor biometric data. The analyte monitoring device of the present invention may usually be used to analyze a specific analyte inside the body or on a surface of the body. For example, the analyte monitoring device of the present invention may have a sensor portion inserted into the skin of a user and may, through the sensor portion, measure glucose etc., in the interstitial fluid in the user's body.

The analyte monitoring device of the present invention may include a sensor portion that measures an analyte, a signal processing portion that processes data measured by the sensor portion, and a communication portion that transfers the processed signal to another device. In addition, the analyte monitoring device of the present invention may include a housing that stably accommodates the sensor portion, the signal processing portion, and the communication portion.

In the description of the present invention, "upward" indicates away from the skin to which the analyte monitoring device of the present invention is attached. In addition, in the description of the present invention, "downward" indicates toward the skin to which the analyte monitoring device of the present invention is attached.

In the description of the present invention, "horizontal direction" indicates a direction substantially parallel to the skin to which the analyte monitoring device of the present invention is attached. Here, it is assumed that the skin to which the analyte monitoring device is attached is a flat surface. Since a lower surface of the housing of the analyte monitoring device of the present invention comes into close contact with the skin, "horizontal direction" may indicate a direction parallel to the lower surface of the housing.

Hereinafter, the present invention will be described with reference to the accompanying drawings.

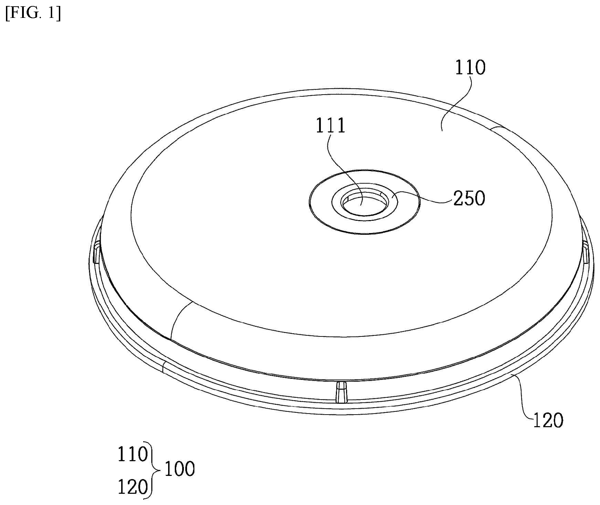

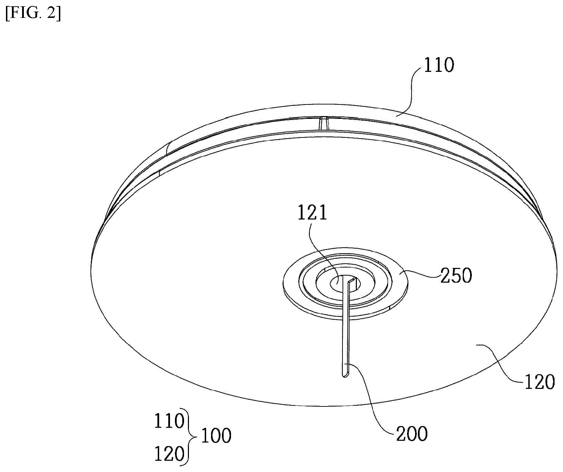

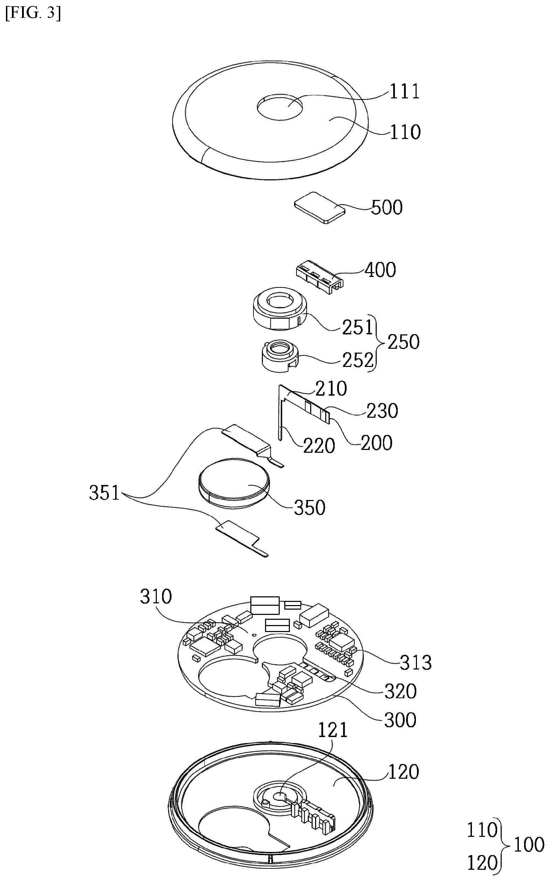

FIG. 1 is a perspective view of an analyte monitoring device according to one embodiment of the present invention, FIG. 2 is a bottom perspective view of the analyte monitoring device according to one embodiment of the present invention, and FIG. 3 is an exploded perspective view of the analyte monitoring device according to one embodiment of the present invention.

Referring to FIGS. 1 to 3, an analyte monitoring device according to the present embodiment may include a housing 100, a sensor portion 200, a circuit portion 300, a contact maintaining portion 400, and a coupling maintaining portion 500.

The housing 100 may include an upper housing 110 and a lower housing 120. The housing 100 may form an inner space between the upper housing 110 and the lower housing 120. Components such as the sensor portion 200, the circuit portion 300, the contact maintaining portion 400, and the coupling maintaining portion 500 may be accommodated in the inner space.

The upper housing 110 and the lower housing 120 may be coupled to each other at a side surface portion of the analyte monitoring device and may form the inner space. The upper housing 110 and the lower housing 120 may be water-tightly coupled so that the inner space is formed to be waterproof from the outside.

The upper housing 110 may include an upper surface and a side surface extending downward from an edge portion of the upper surface. The upper surface of the upper housing 110 may correspond to an upper surface of the analyte monitoring device. The side surface of the upper housing 110 may be coupled to the lower housing 120.

An upper opening 111 may be formed in the upper surface of the upper housing 110. A fixing portion 250 that fixes the sensor portion 200 may be coupled to the upper opening 111. The fixing portion 250 may be coupled to the sensor portion 200 and may prevent the sensor portion 200 from moving.

The fixing portion 250 may include a holder 251 and a packing holder 252. The holder 251 may be coupled to the sensor portion 200 and may fix the sensor portion 200. The packing holder 252 may be coupled to an inner portion of the holder 251. The packing holder 252 may be formed with a width corresponding to a width of the sensor portion 200 and may fix the sensor portion 200. In addition, a needle embedded in an applicator (a device for attaching the analyte monitoring device to the user's skin) may be passed and pressed through the packing holder 252.

The lower housing 120 may include a lower surface and a side surface extending upward from an edge portion of the lower surface. The lower surface of the lower housing 120 may correspond to a lower surface of the analyte monitoring device and may come into contact with the user's skin. The analyte monitoring device may be attached to the user's skin by adhesive tape or an adhesive being attached or applied on the lower surface of the lower housing 120.

A lower opening 121 may be formed in the lower housing 120. The lower opening 121 may be formed at a position facing the upper opening 111 in the up-down direction. When the sensor portion 200 is coupled to the lower housing 120, the sensor portion 200 may pass through the lower opening 121, and a sensing portion 220 of the sensor portion 200 may be exposed to the outside.

One portion of the sensor portion 200 may be located in the inner space of the housing 100, and the other portion thereof may be exposed to the outside through the lower opening 121. The sensor portion 200 may measure an analyte inside the body and may transfer measurement-related data to the circuit portion 300.

The sensor portion 200 may include a connecting portion 210 and the sensing portion 220. The connecting portion 210 may be located in the inner space of the housing 100. The sensing portion 220 may extend from the connecting portion 210 and may be exposed to the outside of the housing 100. The sensor portion 200 may measure an analyte inside the body through the sensing portion 220.

The circuit portion 300 may be located in the inner space of the housing 100. The circuit portion 300 may serve to process data measured through the sensor portion 200 and send the processed data to another device.

The circuit portion 300 may include a body portion 310 and a contact portion 320. Various circuit elements 313 related to processing data and sending the processed data to another device may be mounted on the body portion 310. In addition, a battery 350 for driving such circuit elements 313 may be coupled to the body portion 310.

The battery 350 may be connected to the circuit portion 300 through a battery connecting member 351. The battery connecting member 351 may be coupled to the battery 350 and the circuit portion 300 and may supply power of the battery 350 to the circuit portion 300.

The contact portion 320 may extend from the body portion 310. The contact portion 320 may be in contact with the connecting portion 210. The contact portion 320 may be formed of a flexible material and may be formed to extend in the horizontal direction from a portion adjacent to the body portion 310 and then bend upward or downward due to contact with the connecting portion 210.

The circuit portion 300 may be formed of various materials and may be formed in various shapes.

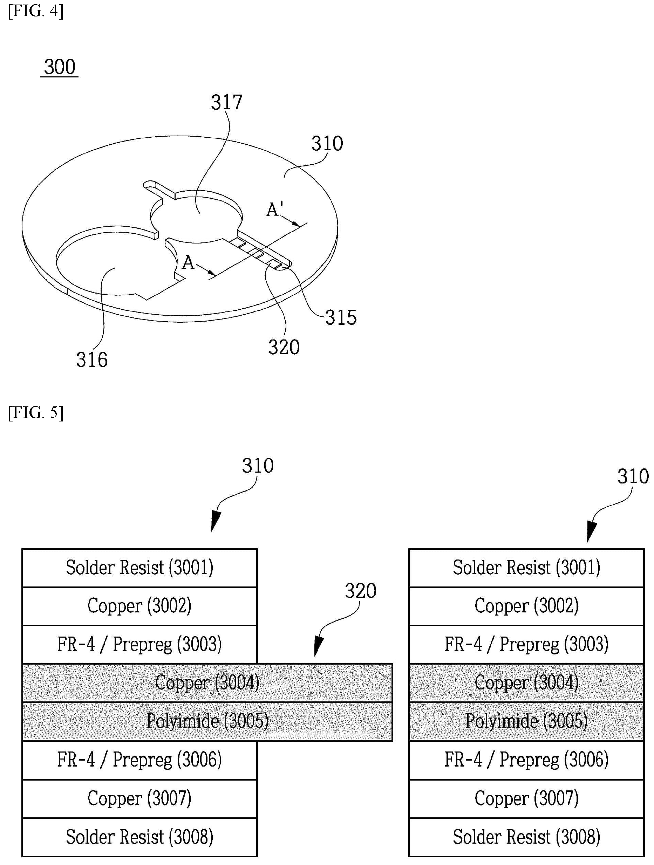

According to one embodiment of the present invention, the circuit portion 300 may be formed as a rigid flexible printed circuit board (PCB). Specifically, the body portion 310 may correspond to the rigid portion of the rigid flexible PCB, and the contact portion 320 may correspond to the flexible portion of the rigid flexible PCB. This embodiment will be described in detail below with reference to FIGS. 4 to 13.

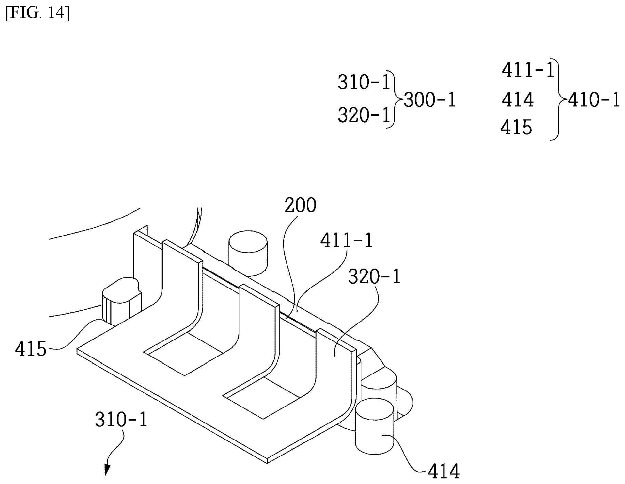

According to another embodiment of the present invention, the circuit portion 300 may be formed in a form in which a rigid PCB and a flexible PCB are coupled. Specifically, the body portion 310 may be formed as a rigid PCB, and the contact portion 320 may be formed as a flexible PCB coupled to the body portion 310. This embodiment will be described in detail below with reference to FIGS. 14 to 16.

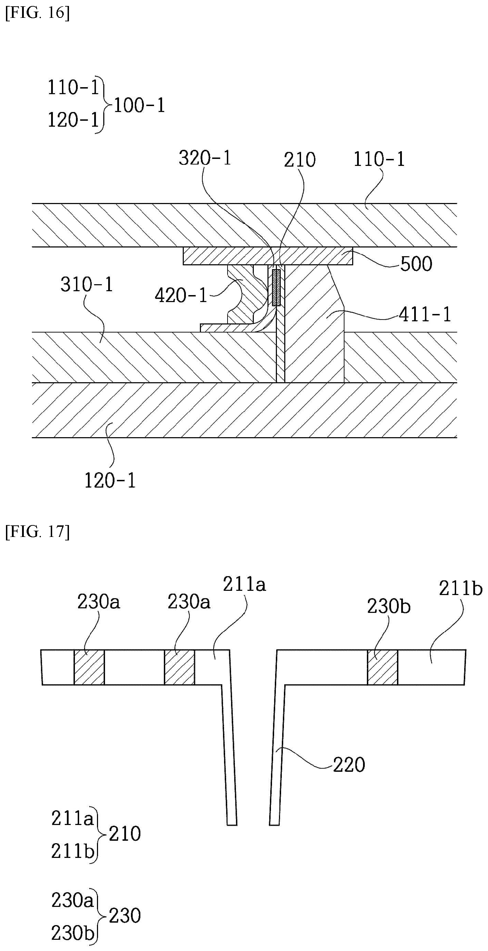

According to still another embodiment of the present invention, the circuit portion 300 may be formed as a flexible PCB. Specifically, the body portion 310 and the contact portion 320 may be integrally formed and may constitute a single flexible PCB. This embodiment will be described in detail below with reference to FIGS. 17 to 22.

The contact maintaining portion 400 may be located in the inner space of the housing 100. The contact maintaining portion 400 may maintain contact between the sensor portion 200 and the contact portion 320. Specifically, the contact maintaining portion 400 may maintain the contact state by bringing the connecting portion 210 of the sensor portion 200 and the contact portion 320 in close contact in directions facing each other.

The coupling maintaining portion 500 may be located in the inner space of the housing 100. The coupling maintaining portion 500 may strengthen the contact state between the sensor portion 200 and the contact portion 320 by maintaining the coupling state of the contact maintaining portion 400.

Hereinafter, the analyte monitoring device according to one embodiment of the present invention will be described with reference to FIGS. 4 to 13.

FIG. 4 is a perspective view of the circuit portion 300 according to one embodiment of the present invention.

Referring to FIG. 4, the circuit portion 300 may include the body portion 310 and the contact portion 320. Although the circuit portion 300 is illustrated as having a circular shape in FIG. 4, the present invention is not limited thereto, and the circuit portion 300 may be formed in various other shapes.

Various configurations may be included in the body portion 310. Specifically, the body portion 310 may include a battery accommodating groove 316 in which at least a portion of the battery 350 is accommodated. In addition, the body portion 310 may include a fixing portion coupling groove 317 to which the fixing portion 250 is coupled.

A groove portion 315 in which the sensor portion 200 is accommodated may be formed in the body portion 310. A portion of the connecting portion 210 of the sensor portion 200 may be accommodated in the groove portion 315, and the connecting portion 210 may be in contact with the contact portion 320.

The contact portion 320 may extend in the horizontal direction in the groove portion 315 of the body portion 310. Contact portions 320 may be formed as many as sensor terminals 230 of the sensor portion 200. Referring to FIG. 4, three contact portions 320 being formed to be spaced from one another is illustrated.

As illustrated in FIG. 4, a plurality of contact portions 320 may extend from one side of the groove portion 315 of the body portion 310. However, in some cases, the plurality of contact portions 320 may extend from both facing sides of the groove portion 315

The contact portions 320 may be formed of a flexible material. Therefore, the contact portions 320 may be deformed due to contact with the connecting portion 210. FIG. 4 illustrates the shape of the contact portions 320 in a state before contact with the connecting portion 210. In the state before contact with the connecting portion 210, the contact portions 320 may extend in the horizontal direction from portions adjacent to the body portion 310. In some cases, in the state before contact with the connecting portion 210, a portion of each contact portion 320 may be bent downward due to the weight of each contact portion 320, but the contact portions 320 may generally remain extended in the horizontal direction.

FIG. 5 is a cross-sectional view schematically illustrating a stacked structure of the circuit portion 300 along line AA' of FIG. 4. A configuration of the circuit portion 300 formed as a rigid flexible PCB will be described in detail with reference to FIG. 5. FIG. 5 is an illustrative drawing for describing the configuration of the circuit portion 300. A stacked form of interconnect layers and resin layers of the circuit portion 300 is not limited to that illustrated in FIG. 5.

The body portion 310 corresponds to a rigid portion of the rigid flexible PCB. The body portion 310 is formed in a form in which a plurality of layers are stacked. Specifically, interconnect layers 3002, 3004, and 3007, resin layers 3003, 3005, and 3006, and solder resist layers 3001 and 3008 may be stacked in the body portion 310. The body portion 310 is formed by stacking rigid interconnect layers 3002 and 3007 and rigid resin layers 3003 and 3006 and thus may be formed as a rigid board as a whole.

The contact portions 320 may correspond to flexible portions of the rigid flexible PCB. The contact portions 320 may extend from portions of the body portion 310. The contact portions 320 may extend from some layers of the plurality of stacked layers constituting the body portion 310.

Specifically, the contact portions 320 may be formed by extending from an intermediate interconnect layer 3004 and the resin layer 3005 of the body portion 310. Here, the intermediate interconnect layer 3004 may be formed relatively thinner than the other interconnect layers 3002 and 3007 and may have a flexible characteristic. In addition, the resin layer 3005 may be formed of a flexible resin material (polyimide., etc.) unlike the other resin layers 3003 and 3006 and may have a flexible characteristic. Therefore, the contact portions 320 may each be formed as a flexible board as a whole.

Although not illustrated in the drawings, in some cases, a non-conductive coverlay layer (not illustrated) etc., may be added on a surface of the interconnect layer 3004 of each contact portion 320.

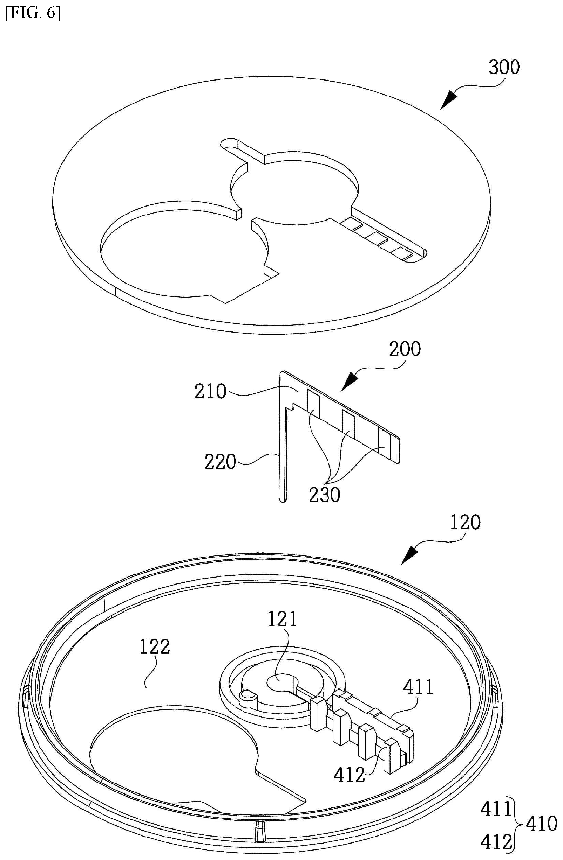

FIG. 6 is an exploded perspective view of the lower housing 120, the sensor portion 200, and the circuit portion 300 according to one embodiment of the present invention.

An extending portion 410 may be formed to extend upward from a lower surface 122 of the lower housing 120. The extending portion 410 may include a protruding portion 411 and a spaced portion 412 that protrude while spaced from each other.

The protruding portion 411 may be formed to protrude upward from the lower surface 122. Specifically, the protruding portion 411 may be formed in the form of a wall that protrudes upward from the lower surface 122. The connecting portion 210 of the sensor portion 200 may be in contact with one surface of the protruding portion 411. One surface of the connecting portion 210 may be in contact with and supported by the protruding portion 411.

The spaced portion 412 may be formed to protrude upward from the lower surface 122. The spaced portion 412 may be located at a position spaced from the protruding portion 411. The protruding portion 411 and the spaced portion 412 may be formed to be spaced at a predetermined interval. The connecting portion 210 of the sensor portion 200 is coupled between the protruding portion 411 and the spaced portion 412.

The spaced portion 412 may be formed as a plurality of spaced portions 412. Referring to FIG. 6, four spaced portions 412 are located to be spaced at predetermined intervals from one another. The contact portions 320 may pass between adjacent spaced portions 412.

The sensor portion 200 may include the connecting portion 210 and the sensing portion 220.

The connecting portion 210 may be located in the inner space of the housing 100. Specifically, the connecting portion 210 may be formed in the form of a plate that stands upright in a vertical direction in the inner space of the housing 100. The connecting portion 210 may include at least one sensor terminal 230. Referring to FIG. 6, three sensor terminals 230 being formed on one surface of the connecting portion 210 is illustrated.

The sensing portion 220 extends from the connecting portion 210. The sensing portion 220 may be bent and extend downward from an end of the connecting portion 210. Accordingly, the sensing portion 220 may extend toward the user's skin.

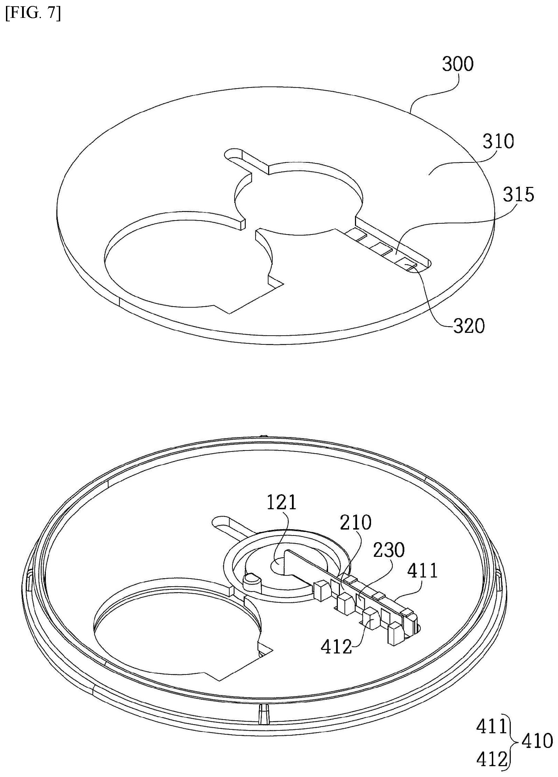

FIG. 7 is a perspective view of a state in which the lower housing 120 and the sensor portion 200 are coupled in the state of FIG. 6.

As illustrated in FIG. 7, the sensor portion 200 may be coupled to the lower housing 120 prior to the circuit portion 300. When the sensor portion 200 is coupled to the lower housing 120, the connecting portion 210 may be fitted between the protruding portion 411 and the spaced portions 412, and the connecting portion 210 having a plate shape may stand upright in the vertical direction. In addition, the sensor terminals 230 are exposed sideward through spaces between the plurality of spaced portions 412. When the sensor portion 200 is coupled to the lower housing 120, the sensing portion 220 may pass through the lower opening 121, and at least a portion of the sensing portion 220 may be exposed to the outside of the housing 100.

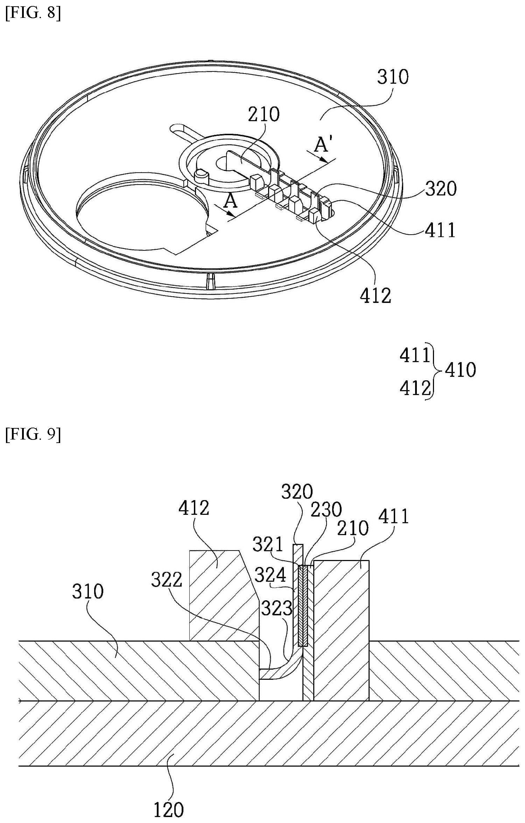

FIG. 8 is a perspective view of a state in which the circuit portion 300 is additionally coupled to the lower housing 120 and the sensor portion 200 in the state of FIG. 7.

Referring to FIG. 8, the circuit portion 300 may be additionally coupled after the lower housing 120 and the sensor portion 200 are coupled. In a state in which the lower housing 120 and the sensor portion 200 are coupled, the circuit portion 300 may be coupled by being moved downward from above the lower housing 120. Accordingly, the sensor portion 200 and the protruding portion 411 are located in the groove portion 315 of the circuit portion 300. In addition, the plurality of contact portions 320 may pass between the plurality of spaced portions 412.

As the circuit portion 300 is coupled, the contact portions 320 come into contact with the connecting portion 210. Specifically, contact terminals 321 formed on surfaces of the contact portions 320 come into contact with the sensor terminals. As the contact portions 320 come into contact with the connecting portion 210, the contact portions 320 may be deformed according to contact. Specifically, the contact portions 320 may be deformed to be bent upward.

Although not illustrated in the drawings, contrary to the above description, in some cases, the circuit portion 300 may be coupled to the lower housing 120 prior to the sensor portion 200, and then the sensor portion 200 may be additionally coupled. In such a case, as the contact portions 320 come into contact with the connecting portion, the contact portions 320 may be deformed to be bent downward.

FIG. 9 is a cross-sectional view of the lower housing 120, the sensor portion 200, and the circuit portion 300 along line AA' of FIG. 8.

The connecting portion 210 of the sensor portion 200 may be fitted between the protruding portion 411 and the spaced portions 412, and the connecting portion 210 having a plate shape may stand upright in the vertical direction. A sensor terminal may be formed on one surface of the connecting portion 210. The other surface of the connecting portion 210 may be in contact with and supported by the protruding portion 411.

The contact portions 320 may each include a horizontal extending portion 322, a bending portion 323, and a contact extending portion 324. The horizontal extending portion 322 may be a portion that is adjacent to the body portion 310 and extends in the horizontal direction. The bending portion 323 may extend from an end of the horizontal extending portion 322 and may bend upward or downward due to contact with the connecting portion 210. The contact extending portion 324 may extend from an end of the bending portion 323 and may extend parallel to the connecting portion 210 while in contact therewith.

The contact portions 320 may each include the contact terminal 321. The contact terminal 321 may be formed on a lower surface of the contact extending portion 324. The contact terminal 321 may come into contact with the sensor terminal 230 corresponding thereto.

The contact portions 320 may be formed of a material that is flexible and has a predetermined restoring force. Therefore, the contact portions 320 may have a property of tending to be restored in the horizontal direction despite being bent upward. Due to such a restoring force, the contact terminal 321 may be in close contact with sensor terminal 230, and the contact state may be maintained.

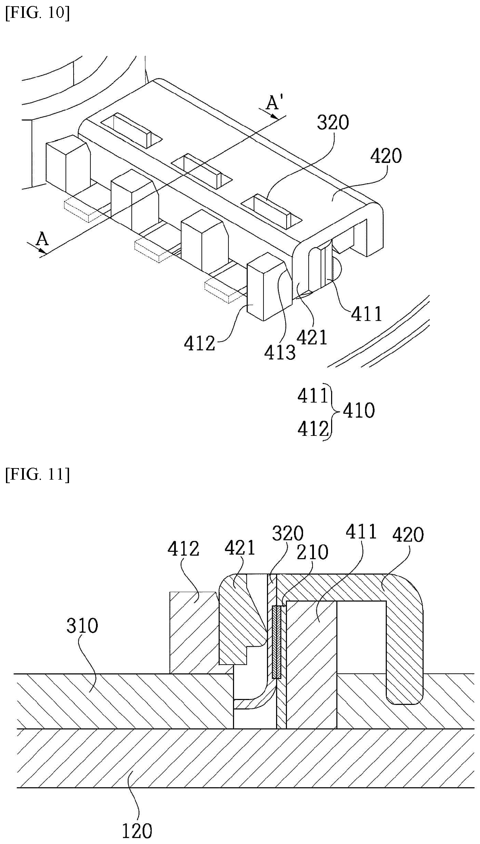

FIG. 10 is a perspective view of a state in which the contact maintaining portion 400 is additionally coupled in the state of FIG. 8. FIG. 11 is a cross-sectional view of the lower housing 120, the sensor portion 200, the circuit portion 300, and the contact maintaining portion 400 along line AA' of FIG. 10.

Referring to FIGS. 10 and 11, the contact maintaining portion 400 may be additionally coupled to maintain the contact state between each contact portion 320 and the connecting portion 210.

The contact maintaining portion 400 may include the extending portion 410 and a coupling portion 420. The extending portion 410 is a configuration extending from the lower housing 120 and has been described above with reference to FIG. 6. The extending portion 410 may include the protruding portion 411 and the spaced portions 412.

The coupling portion 420 is a configuration coupled to the extending portion 410. The coupling portion 420 may be formed as a separate configuration from the housing 100 and may be additionally coupled after the sensor portion 200 and the circuit portion 300 are coupled to the lower housing 120. In one example, the contact maintaining portion 400 may be formed of the protruding portion 411, the spaced portions 412, and a fitting clip coupled from above between the protruding portion 411 and the spaced portions 412.

Specifically, the coupling portion 420 may include an insertion portion 421 that is coupled by being inserted between the protruding portion 411 and the spaced portions 412. When the insertion portion 421 is inserted between the protruding portion 411 and the spaced portions 412, the contact portions 320 and the connecting portion 210 are located between the insertion portion 421 and the protruding portion 411. The contact portions 320 and the connecting portion 210 may remain in close contact with each other between the insertion portion 421 and the protruding portion 411.

The insertion portion 421 may be fitted by being forcibly fitted between the protruding portion 411 and the spaced portions 412. Specifically, a thickness of the insertion portion 421 may be formed to be larger than a space between the contact portions 320 and the spaced portions 412, and the insertion portion 421 may be fitted as a portion between the protruding portion 411 and the spaced portions 412 widens due to the insertion portion 421 being forcibly fitted. In this case, due to a restoring force of the protruding portion 411 and the spaced portions 412, the insertion portion 421 may allow the contact portions 320 to come into close contact with the connecting portion 210 in the horizontal direction toward the connecting portion 210. Accordingly, the close contact state between the contact portions 320 and the connecting portion 210 may be more firmly maintained.

The spaced portions 412 may each include an inclined portion 413 formed in a direction facing the protruding portion 411. When the coupling portion 420 is coupled to the spaced portions 412, the coupling portion 420 may be coupled by being moved downward along the inclined portions 413.

Due to an angle of the inclined portions 413, a self-locking effect of blocking uncoupling of the coupling portion 420 due to an external force may be generated. In this way, the degree to which the connecting portion 210 and the contact portion 320 come into close contact with each other may be improved. When the contact portion 320 includes the plurality of contact portions 320 spaced from one another, the spaced portions 412 may be located between the plurality of contact portions 320.

In FIGS. 10 and 11, the connecting portion 210 and the contact portion 320 are illustrated as being in close contact with each other while the connecting portion 210 is in contact with the protruding portion 411, and the contact portion 320 is in contact with the coupling portion 420. However, conversely, the connecting portion 210 and the contact portion 320 may be in close contact with each other while the contact portion 320 is in contact with the protruding portion 411, and the connecting portion 210 is in contact with the coupling portion 420.

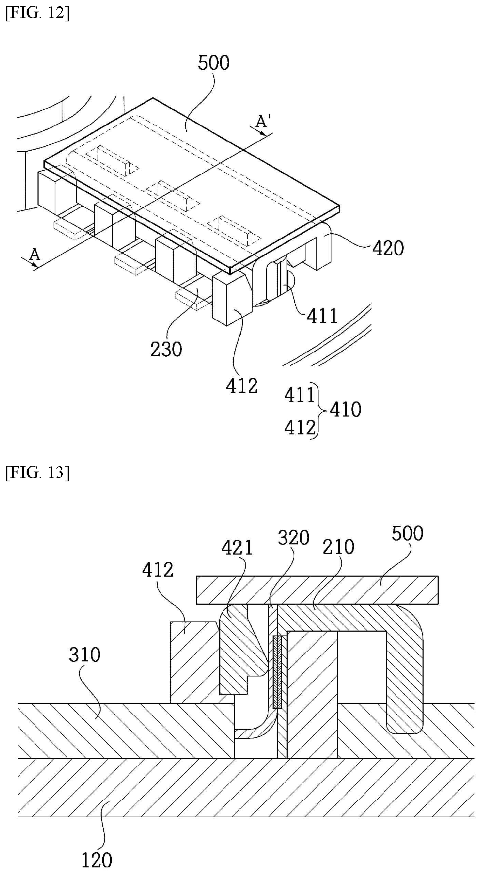

FIG. 12 is a perspective view of a state in which the coupling maintaining portion 500 is additionally coupled in the state of FIG. 10. FIG. 13 is a cross-sectional view of the lower housing 120, the sensor portion 200, the circuit portion 300, the contact maintaining portion 400, and the coupling maintaining portion 500 along line AA' of FIG. 12.

Referring to FIGS. 12 and 13, the coupling maintaining portion 500 may be additionally coupled to maintain the coupling state of the coupling portion 420.

The coupling maintaining portion 500 may maintain the coupling state between the extending portion 410 and the coupling portion 420. Specifically, the coupling maintaining portion 500 may be located between the upper housing 110 and the contact maintaining portion 400 to prevent the coupling portion 420 from moving upward and being detached from the extending portion 410.

The coupling maintaining portion 500 may include a flexible member located between a lower surface of the upper housing 110 and the contact maintaining portion 400. For example, the flexible member may be cushion tape, foam tape, Poron tape, or the like.

The coupling maintaining portion 500 may be located on an upper surface of the coupling portion 420. Specifically, the coupling maintaining portion 500 may be located in a space between the upper housing 110 and the coupling portion 420. When the upper housing 110 is coupled to the lower housing 120, the coupling maintaining portion 500 may be compressed between the upper housing 110 and the coupling portion 420. Accordingly, the coupling maintaining portion 500 may allow the coupling portion 420 to come into close contact with the extending portion 410.

The coupling maintaining portion 500 may be attached to a lower side surface of the upper surface of the upper housing 110. As the upper housing 110 is coupled to the lower housing 120 in a state in which the coupling maintaining portion 500 is attached to the upper housing 110, the coupling maintaining portion 500 may be located in a space between the upper housing 110 and the coupling portion 420. To this end, a position at which the coupling maintaining portion 500 will be attached may be specified on the lower side surface of the upper surface of the upper housing 110.

Hereinafter, an analyte monitoring device according to another embodiment of the present invention will be described with reference to FIGS. 14 to 16.

FIG. 14 is a perspective view of a state in which a lower housing 120-1, a sensor portion 200, and a circuit portion 300-1 are coupled according to another embodiment of the present invention.

The circuit portion 300-1 may be formed in a form in which a rigid PCB and a flexible PCB are coupled. Specifically, a body portion 310-1 may be formed as a rigid PCB, and a contact portion 320-1 may be formed as a flexible PCB coupled to the body portion 310-1.

The contact portion 320-1 may be coupled to an upper surface of the body portion 310-1. The contact portion 320-1 may be coupled to be electrically connected to the body portion 310-1, and measurement data of the sensor portion 200 may be transferred to the body portion 310-1 through the contact portion 320-1.

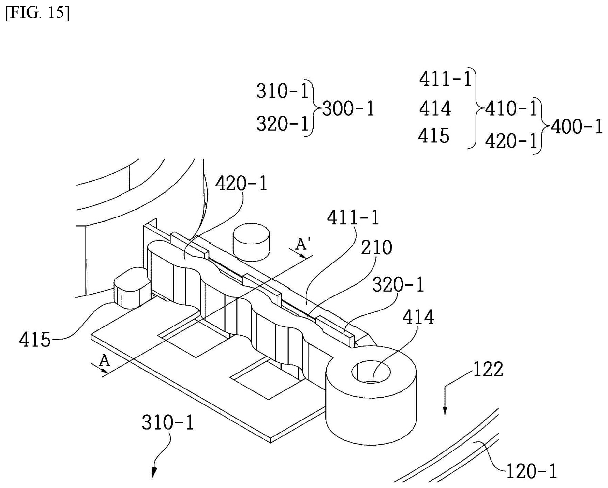

FIG. 15 is a perspective view of a state in which a contact maintaining portion 400-1 is additionally coupled in the state of FIG. 14. FIG. 16 is a cross-sectional view of the lower housing 120-1, the sensor portion 200, the circuit portion 300-1, and the contact maintaining portion 400-1 along line AA' of FIG. 15.

Referring to FIGS. 15 and 16, the contact maintaining portion 400-1 may be additionally coupled to maintain a contact state between the contact portion 320-1 and a connecting portion 210.

The contact maintaining portion 400-1 may include an extending portion 410-1 and a coupling portion 420-1.

The extending portion 410-1 may include a protruding portion 411-1, a rotating shaft 414, and a fixing pin 415.

The protruding portion 411-1 may be formed in the form of a wall that protrudes upward from a lower surface 122. The connecting portion 210 of the sensor portion 200 may be in contact with one surface of the protruding portion 411-1. One surface of the connecting portion 210 may be in contact with and supported by the protruding portion 411-1.

The rotating shaft 414 may be formed in the form of a column that protrudes upward from the lower surface 122. The rotating shaft 414 may be located at a position spaced from the protruding portion 411-1.

The coupling portion 420-1 may be a rotary member that can rotate about the rotating shaft 414. The coupling portion 420-1 may include an insertion hole into which the rotating shaft 414 is inserted. The coupling portion 420-1 may rotate and may move to be adjacent to the protruding portion 411-1.

The fixing pin 415 may fix the coupling portion 420-1 in a state in which the coupling portion 420-1 is adjacent to the protruding portion 411-1. Specifically, the coupling portion 420-1 may rotate to be adjacent to the protruding portion 411-1 and may be fitted to the fixing pin 415. Due to the fixing pin 415, the coupling portion may be prevented from rotating away from the protruding portion 411-1.

When the coupling portion 420-1 and the protruding portion 411-1 are fixed while adjacent to each other, the contact portion 320-1 and the connecting portion 210 may remain in close contact with each other between the coupling portion 420-1 and the protruding portion 411-1. As the coupling portion 420-1 is fixed by the fixing pin 415, the coupling portion 420-1 may allow the contact portion 320-1 to come into close contact with the connecting portion 210 in the horizontal direction toward the connecting portion 210. Accordingly, the close contact state between the contact portion 320-1 and the connecting portion 210 may be more firmly maintained.

Referring to FIG. 16, the coupling portion 420-1 may come into contact with the contact portion 320-1 and may allow the connecting portion 210 and the contact portion 320-1 to come into close contact with each other. In some cases, as illustrated in FIG. 16, the coupling portion 420-1 may be formed to have at least one portion protruding toward the contact portion 320-1. However, the coupling portion 420-1 is not limited to the shape illustrated in FIG. 16 and may be embodied in various other shapes.

A coupling maintaining portion 500-1 may be located in an inner space of a housing 100-1. The coupling maintaining portion 500-1 may be attached to a lower side surface of an upper surface of an upper housing 110-1. As the upper housing 110-1 is coupled to the lower housing 120-1 in a state in which the coupling maintaining portion 500-1 is attached to the upper housing 110-1, the coupling maintaining portion 500-1 may be located in a space between the upper housing 110-1 and the coupling portion 420-1. To this end, a position at which the coupling maintaining portion 500-1 will be attached may be specified on the lower side surface of the upper surface of the upper housing 110-1.

Hereinafter, an analyte monitoring device according to still another embodiment of the present invention will be described with reference to FIGS. 17 to 22.

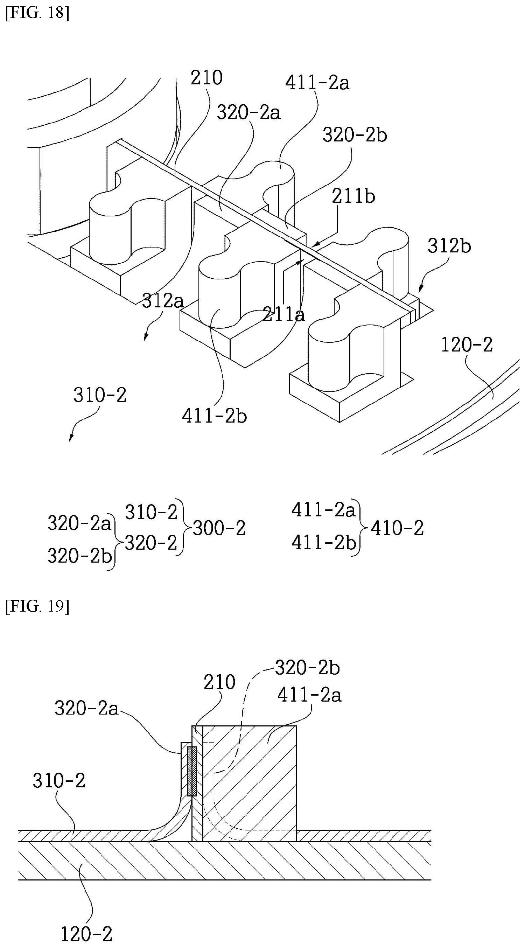

FIG. 17 is a view separately illustrating either surface of the sensor portion 200 according to still another embodiment of the present invention. FIG. 18 is a perspective view of a state in which a lower housing 120-2, the sensor portion 200, and a circuit portion 300-2 are coupled according to still another embodiment of the present invention. FIG. 19 is a cross-sectional view along line AA' of FIG. 18.

Referring to FIG. 17, sensor terminals 230 may be formed on a first surface 211a or a second surface 211b of a connecting portion 210. Here, the first surface 211a and the second surface 211b may be opposite surfaces of the connecting portion 210. As illustrated in FIG. 17, two first sensor terminals 230a may be formed on the first surface 211a of the connecting portion 210, and one second sensor terminal 230b may be formed on the second surface 211b opposite to the first surface 211a. The number of first sensor terminals 230a and the number of second sensor terminals 230b may vary.

The first sensor terminal 230a and the second sensor terminal 230b may be formed on different potions of the sensor portion 200. Specifically, the first sensor terminal 230a may be formed on the first surface 211a of a first portion of the connecting portion, and the second sensor terminal 230b may be formed on the second surface 211b of a second portion of the connecting portion 210. Here, the first portion and the second portion of the connecting portion 210 may be portions that do not face each other. Therefore, the first sensor terminal 230a and the second sensor terminal 230b may be located to not face each other on the connecting portion 210.

Referring to FIG. 18, the circuit portion 300-2 may be formed as a flexible PCB. Specifically, the circuit portion 300-2 may be a single flexible PCB on which a body portion 310-2 and contact portions 320-2 are integrally formed.

The contact portions 320-2 may be formed to correspond to the above-described sensor terminals 230 of the sensor portion 200. Specifically, the contact portions 320-2 may include a first contact portion 320-2a and a second contact portion 320-2b. The first contact portion 320-2a may extend from a first end 312a of the body portion 310-2 toward the first surface 211a and may come into contact with the first sensor terminal 230a. The second contact portion 320-2b may extend from a second end 312b of the body portion 310-2 toward the second surface 211b and may come into contact with the second sensor terminal 230b.

Referring to FIGS. 18 and 19, extending portions 410-2 may include protruding portions that protrude from the lower housing 120-2. The protruding portions may include a first protruding portion 411-2a and a second protruding portion 411-2b that are spaced from each other.

The first protruding portion 411-2a may come into contact with the second surface 211b of the first portion of the connecting portion 210. The first sensor terminal 230a may be formed on the first surface 211a of the first portion of the connecting portion 210. Therefore, the first protruding portion 411-2a may support the opposite surface of the first sensor terminal 230a.

The second protruding portion 411-2b may come into contact with the first surface 211a of the second portion of the connecting portion 210. The second sensor terminal 230b may be formed on the second surface 211b of the second portion of the connecting portion 210. Therefore, the second protruding portion 411-2b may support the opposite surface of the second sensor terminal 230b.

As illustrated in FIG. 18, a plurality of first protruding portions 411-2a and a plurality of second protruding portions 411-2b may be formed. Therefore, when the sensor portion 200 is coupled to the lower housing 120-2, the second sensor terminal 230b may be exposed between the plurality of first protruding portions 411-2a, and the first sensor terminal 230a may be exposed between the plurality of second protruding portions 411-2b. Then, when the circuit portion 300-2 is coupled to the lower housing 120-2, the first contact portion 320-2a may pass between the plurality of second protruding portions 411-2b and may come into contact with the first sensor terminal 230a, and the second contact portion 320-2b may pass between the plurality of first protruding portions 411-2a and may come into contact with the second sensor terminal 230b.

The circuit portion 300-2 may be a flexible PCB on which the body portion 310-2 and the contact portions 320-2 are integrally formed. In this case, each contact portion 320-2 may protrude and extend from one portion of the body portion 310-2 in the horizontal direction.

Specifically, when the circuit portion 300-2 is the flexible PCB on which the body portion 310-2 and the contact portions 320-2 are integrally formed, thicknesses of the body portion 310-2 and each contact portion 320-2 may be the same. That is, unlike in FIG. 11 where the contact portion 320 extends between portions of the body portion 310 and has a smaller thickness than (is formed to be thinner than) the body portion 310, the contact portion 320-2 may be integrally formed with the body portion 310-2 (may be formed to have the same thickness as the body portion).

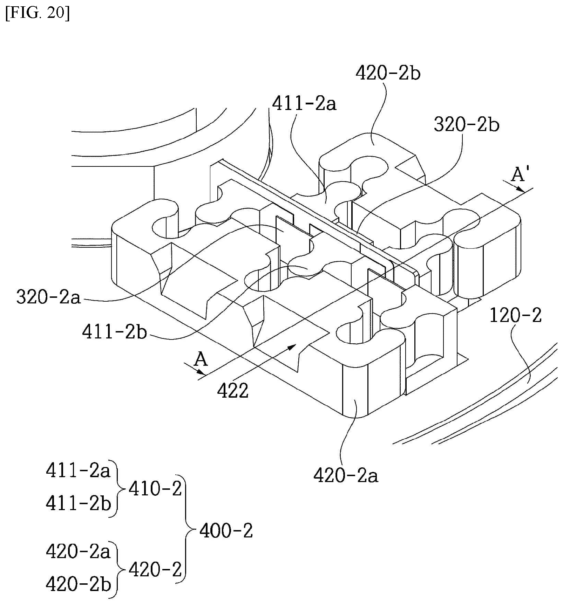

Referring to FIG. 20, a contact maintaining portion 400-2 includes the extending portions 410-2 and coupling portions 420-2. The coupling portions 420-2 may include a first coupling portion 420-2a and a second coupling portion 420-2b.

The first coupling portion 420-2a may be coupled to the second protruding portion 411-2b. Specifically, the first coupling portion 420-2a may be coupled to the second protruding portion 411-2b in a direction toward the first surface 211a. As the first coupling portion 420-2a is coupled to the second protruding portion 411-2b, the first coupling portion 420-2a may allow the first contact portion 320-2a and the first sensor terminal 230a to come into close contact.

As illustrated in FIG. 20, a single first coupling portion 420-2a may be coupled to a plurality of second protruding portions 411-2b. To this end, coupling grooves equal in number to the second protruding portions 411-2b may be formed in the first coupling portion 420-2a.

The second coupling portion 420-2b may be coupled to the first protruding portion 411-2a. Specifically, the second coupling portion 420-2b may be coupled to the first protruding portion 411-2a in a direction toward the second surface 211b. As the second coupling portion 420-2b is coupled to the first protruding portion 411-2a, the second coupling portion 420-2b may allow the second contact portion 320-2b and the second sensor terminal 230b to come into close contact.

As illustrated in FIG. 20, a single second coupling portion 420-2b may be coupled to a plurality of first protruding portions 411-2a. To this end, coupling grooves equal in number to the first protruding portions 411-2a may be formed in the second coupling portion 420-2b.

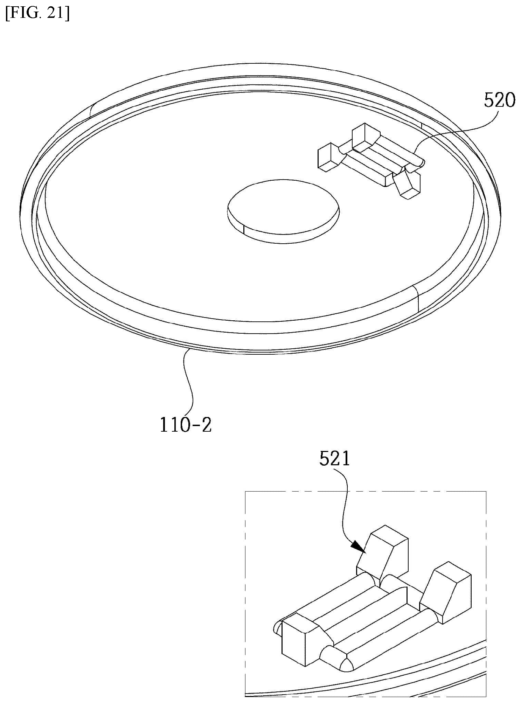

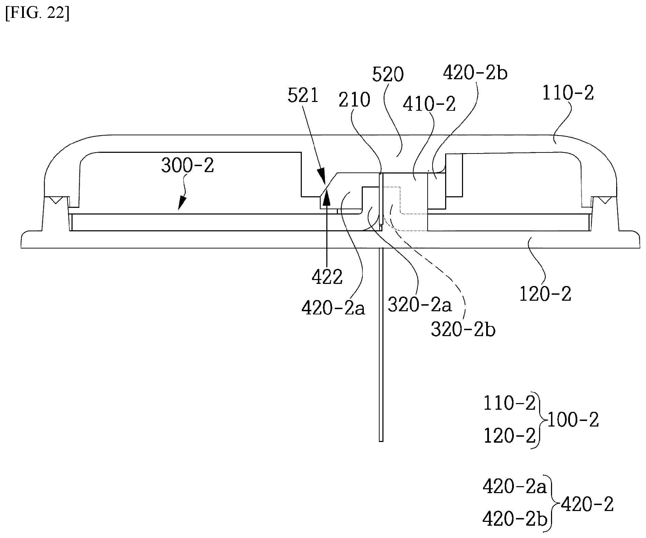

An inclined surface 422 coupled to a coupling maintaining portion 500-2 may be formed on an upper portion of each coupling portion 420-2. Coupling of the inclined surface 422 to the coupling maintaining portion 500-2 will be described below with reference to FIGS. 21 and 22.

FIG. 21 is a perspective view of a lower surface of an upper housing 110-2 according to one embodiment of the present invention, and FIG. 22 is a cross-sectional view of a state in which the lower housing 120-2, the circuit portion 300-2, the contact maintaining portion 400-2, the coupling maintaining portion 500-2, and the upper housing 110-2 are coupled according to one embodiment of the present invention.

Referring to FIG. 21, a housing 100-2 may include the coupling maintaining portion 500-2. In one example, the coupling maintaining portion 500-2 may include an upper protruding portion 520 that protrudes from a lower surface of the upper housing 110-2 and comes into contact with the coupling portion 420-2. Specifically, the upper protruding portion 520 may be formed in a form that comes into contact with the coupling portion 420-2 by protruding from the lower surface of the upper housing 110-2.

The upper protruding portion 520 may include a facing inclined surface 521. In FIG. 20, the coupling portion 420-2 of the contact maintaining portion 400-2 may include the inclined surface 422. Facing inclined surfaces 521 may be formed to be equal in number to the inclined surfaces 422 of the coupling portions 420-2 at positions facing the inclined surfaces 422. By the facing inclined surface 521 coming into contact with the inclined surface 422, the coupling maintaining state between the extending portion 410-2 and the coupling portion 420-2 may be improved.

As illustrated in FIG. 22, in a state in which the sensor portion 200, the circuit portion 300-2, and the contact maintaining portion 400-2 are coupled to the lower housing 120-2, the lower housing 120-2 and the upper housing 110-2 may be coupled. Specifically, the lower housing 120-2 and the upper housing 110-2 may be coupled to be aligned so that the coupling portion 420-2 and the upper protruding portion 520 face each other. When the upper housing 110-2 is moved downward and coupled to the lower housing 120-2, the upper protruding portion 520 and the coupling portion 420-2 are coupled in the inner space.

As the upper housing 110-2 is moved downward, the facing inclined surface 521 may move downward while in contact with the inclined surface 422 and may allow the first coupling portion 420-2a and the second coupling portion 420-2b to come into close contact in directions facing each other.

Specifically, as the lower housing 120-2 and the upper housing 110-2 are coupled, the first coupling portion 420-2a may come into close contact in the direction toward the first surface 211a due to the upper protruding portion 520. Therefore, the close contact state between the first contact portion 320-2a and the first sensor terminal 230a may be further strengthened. In addition, the second coupling portion 420-2b may come into close contact in the direction toward the second surface 211b due to the upper protruding portion 520. Therefore, the close contact state between the second contact portion 320-2b and the second sensor terminal 230b may be further strengthened.

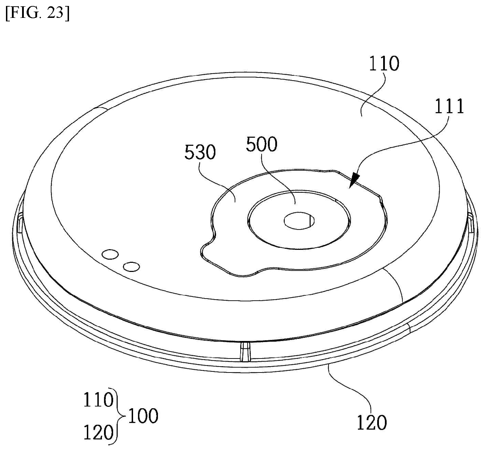

Hereinafter, additional embodiments of the present invention will be described with reference to FIGS. 23 to 39. In the additional embodiments described with reference to FIGS. 23 to 39, when the same reference numerals are used as in the description of the preceding embodiments given with reference to FIGS. 1 to 22, components denoted by the corresponding same reference numerals will be described based on the contents described in the following additional embodiments.

In the description of one additional embodiment, "horizontal direction" indicates a direction substantially parallel to the skin to which the analyte monitoring device of the present invention is attached. Here, it is assumed that the skin to which the analyte monitoring device is attached is a flat surface. Since a lower surface of the housing of the analyte monitoring device of the present invention comes into close contact with the skin, "horizontal direction" may indicate a direction parallel to the lower surface of the housing.

In the description of one additional embodiment, "vertical direction" refers to a direction perpendicular to the horizontal direction and moving away from the skin. Since the lower surface of the housing of the analyte monitoring device of the present invention comes into close contact with the skin, "vertical direction" may indicate a direction perpendicular to the lower surface of the housing.

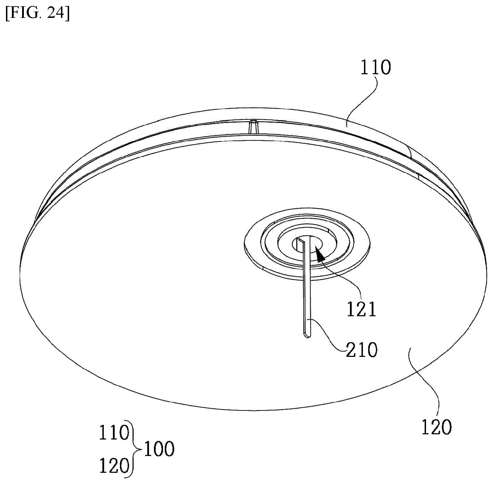

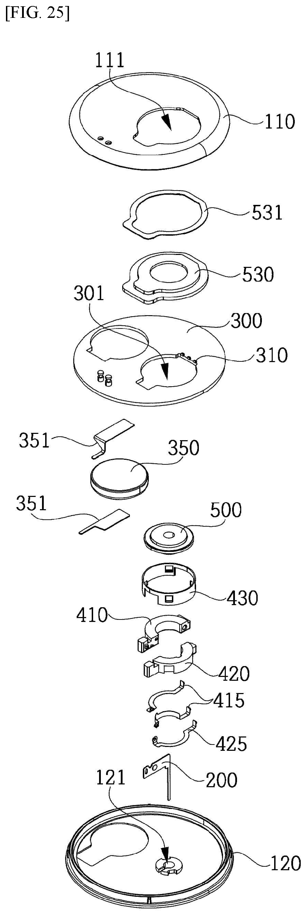

FIG. 23 is a perspective view of an analyte monitoring device according to one additional embodiment of the present invention, FIG. 24 is a bottom perspective view of the analyte monitoring device according to one additional embodiment of the present invention, and FIG. 25 is an exploded perspective view of the analyte monitoring device according to one additional embodiment of the present invention.

Referring to FIGS. 23 to 25, the analyte monitoring device according to the present embodiment may include a housing 100, a sensor portion 200, a circuit portion 300, a sensor holder 400, and a sensor fixing portion 500.

The housing 100 may include an upper housing 110 and a lower housing 120. The housing 100 may form an inner space between the upper housing 110 and the lower housing 120. Components such as the sensor portion 200, the circuit portion 300, the sensor holder 400, and the sensor fixing portion 500 may be accommodated in the inner space.

The upper housing 110 and the lower housing 120 may be coupled to each other at a side surface portion of the analyte monitoring device and may form the inner space. The upper housing 110 and the lower housing 120 may be water-tightly coupled so that the inner space is formed to be waterproof from the outside.

The upper housing 110 may include an upper surface and a side surface extending downward from an edge portion of the upper surface. The upper surface of the upper housing 110 may correspond to an upper surface of the analyte monitoring device. The side surface of the upper housing 110 may be coupled to the lower housing 120.

An upper opening 111 may be formed in the upper surface of the upper housing 110. The sensor fixing portion 500 that fixes the position of the sensor portion 200 in the inner space may be located in the upper opening 111.

The lower housing 120 may include a lower surface and a side surface extending upward from an edge portion of the lower surface. The lower surface of the lower housing 120 may correspond to a lower surface (a bottom surface) of the analyte monitoring device and may come into contact with the user's skin. The analyte monitoring device may be attached to the user's skin by adhesive tape or an adhesive being attached or applied on the lower surface of the lower housing 120.

A through-hole 121 may be formed in the lower housing 120. The through-hole 121 may be formed at a position facing the upper opening 111 in the up-down direction. When the sensor portion 200 is coupled to the lower housing 120, the sensor portion 200 may pass through the through-hole 121, and a sensing portion 220 of the sensor portion 200 may be exposed to the outside.

One portion of the sensor portion 200 may be located in the inner space of the housing 100, and the other portion thereof may be exposed to the outside through the through-hole 121. The sensor portion 200 may measure an analyte inside the body and may transfer measurement-related data to the circuit portion 300.

The sensor portion 200 may include a connecting portion 210 and the sensing portion 220. The connecting portion 210 may be located in the inner space of the housing 100. The sensing portion 220 may extend from the connecting portion 210 and may be exposed to the outside of the housing 100. The sensor portion 200 may measure an analyte inside the body through the sensing portion 220.

The circuit portion 300 may be located in the inner space of the housing 100. The circuit portion 300 may serve to process data measured through the sensor portion 200 and send the processed data to another device.

An opening 301 in which the sensor portion 200 and the sensor holder 400 are accommodated may be formed in the circuit portion 300. A signal terminal 310 electrically connected to signal transfer portions 415 and 425 may be formed at a portion of the circuit portion 300 that is adjacent to the opening 301.

The circuit portion 300 may include a terminal on or to which the sensor portion 200, a battery 350, and a signal processing portion (not illustrated) are mounted or coupled. Specifically, the circuit portion 300 may include the signal terminal 310 formed at a portion adjacent to a contact portion. The signal terminal 310 may be electrically connected to circuit connecting terminals 415b and 425b of the signal transfer portions 415 and 425.

The battery 350 for driving a circuit element or the like may be coupled to the circuit portion 300. The battery 350 may be coupled to the circuit portion 300 through a battery connecting member 351. The battery connecting member 351 may be coupled to the battery 350 and the circuit portion 300 and may supply power of the battery 350 to the circuit portion 300.

The signal processing portion (not illustrated) may be mounted on the circuit portion 300. The signal processing portion may process data sensed by the sensor portion 200. Various elements other than the signal processing portion, such as a communication portion (not illustrated) related to sending the processed data to another device, may be mounted on the circuit portion 300.

The sensor holder 400 may be located in the inner space of the housing 100. The sensor holder 400 may be coupled to the sensor portion 200, may fix the position of the sensor portion 200, and may transfer the data sensed by the sensor portion 200 to the circuit portion 300.

The sensor holder 400 may include a contact portion 401 and a close contact maintaining portion 430.

The contact portion 401 may include a first contact portion 410 and a second contact portion 420. The first contact portion 410 and the second contact portion 420 may be in close contact with the connecting portion 210 of the sensor portion 200 disposed therebetween. Accordingly, the sensor portion 200 may be fixed between the first contact portion 410 and the second contact portion 420.

At least one of the first contact portion 410 and the second contact portion 420 may serve as a transfer contact portion. The transfer contact portion is a configuration that not only comes into physical contact with the sensor portion 200 but also performs a function of transferring data sensed by the sensor portion 200 to the circuit portion 300. A configuration corresponding to the transfer contact portion may include the signal transfer portions 415 and 425. The signal transfer portions 415 and 425 may electrically connect the sensor portion 200 and the circuit portion 300.

The close contact maintaining portion 430 may be coupled to the contact portion 401 and may maintain a state in which the first contact portion 410 and the second contact portion 420 are in close contact with each other. Specifically, the close contact maintaining portion 430 may apply an external force to the first contact portion 410 and the second contact portion 420, and a state in which the connecting portion 210 of the sensor portion 200 is tightly placed between the first contact portion 410 and the second contact portion 420 may be more firmly maintained.

The sensor fixing portion 500 may be located in the inner space of the housing 100. The sensor fixing portion 500 may be coupled to the sensor portion 200 in a sensor accommodating space inside the contact portion 401. The sensor fixing portion 500 may fix the position of the sensor portion 200.

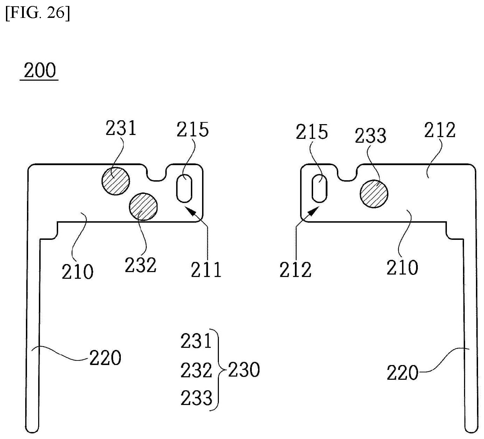

Hereinafter, an analyte monitoring device according to one additional embodiment of the present invention will be described with reference to FIGS. 26 to 35. FIG. 26 is a view separately illustrating either surface of a sensor portion according to one additional embodiment of the present invention.

Referring to FIG. 26, a sensor portion 200 may include a connecting portion 210 and a sensing portion 220. The connecting portion 210 may be located in an inner space of a housing 100. Specifically, the connecting portion 210 may be formed in the form of a plate that stands upright in the vertical direction in the inner space of the housing 100. The connecting portion 210 may include a first surface 211 and a second surface 212 that stand upright in the vertical direction. The first surface 211 and the second surface 212 may correspond to surfaces opposite to each other.

The connecting portion 210 may include at least one sensor terminal 230. The sensor terminal 230 may be formed on at least one of the first surface 211 and the second surface 212. Referring to FIG. 26, two first sensor terminals 231 and 232 being formed on the first surface 211 of the connecting portion 210 and one second sensor terminal 233 being formed on the second surface 212 of the connecting portion 210 is illustrated. Surfaces of the connecting portion 210 where sensor terminals are formed and positions of the sensor terminals may vary.

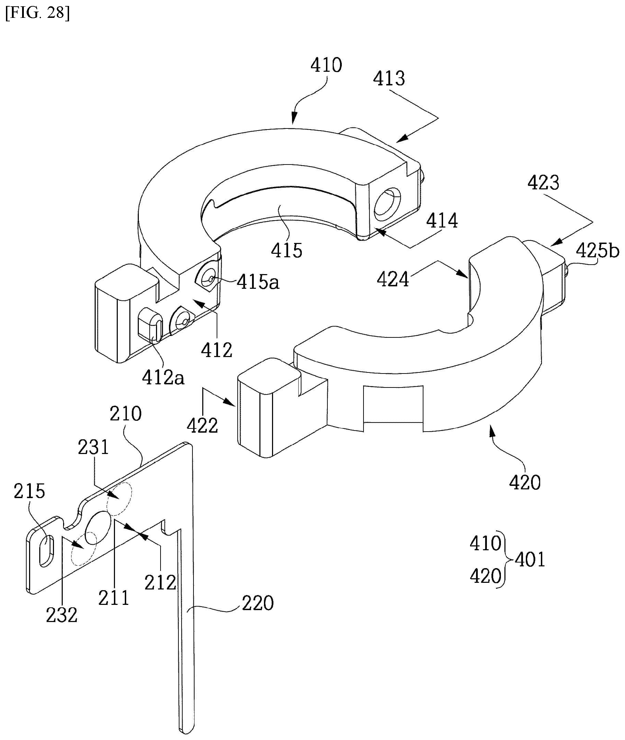

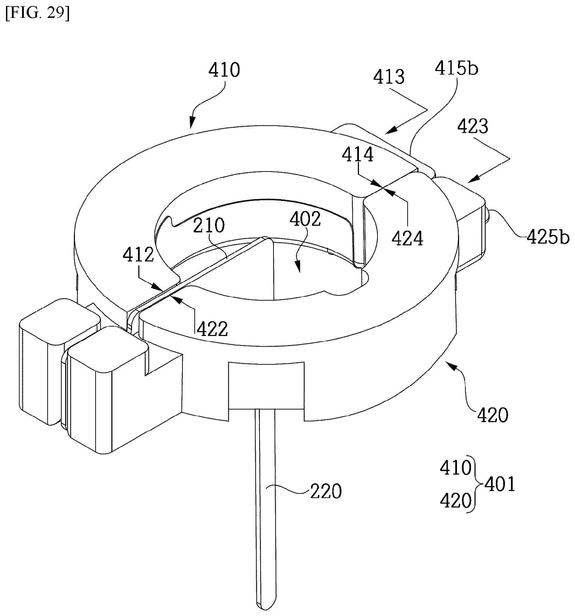

The connecting portion 210 may include a sensor fastening portion 215. The sensor fastening portion 215 may be a configuration for strengthening coupling between the connecting portion 210 and the contact portion 401 and fixing the position of the connecting portion 210 between the first contact portion 410 and the second contact portion 420. The sensor fastening portion 215 may be formed as a groove, an opening, or the like. Fastening portions 412a and 422a of the contact portion 401, which will be described below, may be inserted into and coupled to the sensor fastening portion 215. The form in which the sensor fastening portion 215 and the fastening portions 412a and 422a of the contact portion 401 are coupled will be described in more detail below with reference to FIGS. 28 and 29.

The sensing portion 220 extends from the connecting portion 210. The sensing portion 220 may be bent and extend downward from an end of the connecting portion 210. Accordingly, the sensing portion 220 may extend toward the user's skin.

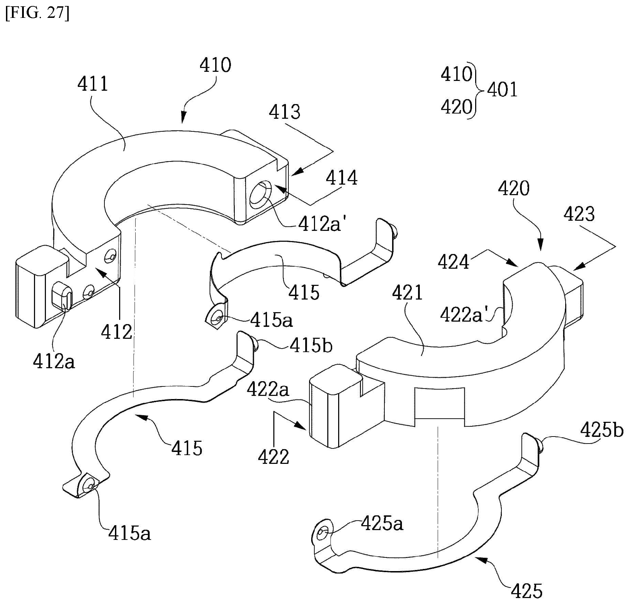

FIG. 27 is an exploded perspective view of a contact portion according to one additional embodiment of the present invention.

Referring to FIG. 27, the contact portion 401 may include the first contact portion 410 and the second contact portion 420 separated from each other. The first contact portion 410 and the second contact portion 420 may be formed as separate members and then be brought into close contact with and coupled to each other with the connecting portion 210 disposed therebetween.

As illustrated in FIG. 27, the contact portion 401 may be formed in a ring shape having a space formed therein. Specifically, the first contact portion 410 and the second contact portion 420 may each be formed in a semicircular shape and then be coupled to each other to form a ring-shaped structure.

At least one of the first contact portion 410 and the second contact portion 420 may serve as a transfer contact portion. The transfer contact portion is a configuration that not only comes into physical contact with the sensor portion 200 but also performs a function of transferring data sensed by the sensor portion 200 to the circuit portion 300. The transfer contact portion may include a signal transfer portion coupled to a contact portion body. The signal transfer portion may be formed of a conductive material and may electrically connect the sensor terminal 230 and the circuit portion 300.

In FIG. 27, the first contact portion 410 and the second contact portion 420 both serving as transfer contact portions is illustrated. However, in some cases, only one of the first contact portion 410 and the second contact portion 420 may serve as a transfer contact portion. In a case in which only the first contact portion 410 serves as a transfer contact portion and the second contact portion 420 does not serve as a transfer contact portion, a signal transfer portion may not be included in the second contact portion 420. In addition, a sensor terminal may not be formed on the second surface 212 of the connecting portion 210 of the sensor portion 200 that comes into contact with the second contact portion 420.

Hereinafter, description will be given based on the case in which both the first contact portion 410 and the second contact portion 420 serve as transfer contact portions as illustrated in FIG. 27.

Referring to FIG. 27, the first contact portion 410 includes a first contact portion body 411 and a first signal transfer portion 415. The first contact portion body 411 may include a first contact surface 412, a first circuit connecting surface 413, and a first coupling surface 414.

The first contact surface 412 corresponds to a surface coming into contact with the connecting portion 210 of the sensor portion 200. The first contact surface 412 may be formed on one end portion of the first contact portion body 411. A sensor connecting terminal 415a of the first signal transfer portion 415 is formed on the first contact surface 412. The sensor connecting terminal 415a of the first signal transfer portion 415 comes into contact with the first sensor terminals 231 and 232 formed on the first surface 211 of the connecting portion 210.

The first circuit connecting surface 413 corresponds to a surface adjacent to the circuit portion 300. The first circuit connecting surface 413 may be formed on the other end portion of the first contact portion body 411. A circuit connecting terminal 415b of the first signal transfer portion 415 is formed on the first circuit connecting surface 413. The first circuit connecting surface 413 may be located to be adjacent to the signal terminal 310 of the circuit portion 300.

The first coupling surface 414 corresponds to a surface that is in contact with and coupled to the second contact portion 420. The first contact portion 410 may be coupled to the second contact portion 420 with the sensor portion 200 disposed therebetween at the first contact surface 412 portion and may be directly coupled to the second contact portion 420 at the first coupling surface 414 portion.

The first signal transfer portion 415 may be coupled to the first contact portion body 411. The first signal transfer portion 415 may include the sensor connecting terminal 415a at one end and the circuit connecting terminal 415b at the other end. The sensor connecting terminal 415a of the first signal transfer portion 415 may be formed on the first contact surface 412, and the circuit connecting terminal 415b may be formed on the first circuit connecting surface 413.

Referring to FIG. 27, two first signal transfer portions 415 being coupled to the first contact portion body 411 is illustrated. The two first signal transfer portions 415 spaced from each other may be respectively in contact with the two first sensor terminals 231 and 232 formed on the first surface 211 of the connecting portion 210.

The first signal transfer portions 415 are formed of a conductive material. Specifically, the first signal transfer portions 415 may be plating layers coupled to the first contact portion body 411. The plating layers may be formed on the first contact portion 410 using various methods. The plating layers may be formed by being selectively plated on the first contact portion body 411 formed of a nonconductive material.

In one example, the first signal transfer portions 415 may be plating layers formed using a laser direct structuring (LDS) method. The LDS method is a laser direct structuring method in which a laser is selectively radiated only on a pattern region where a plating layer is desired to be formed on a base member, and then a plating layer is selectively formed only on the corresponding pattern region.

In the present invention, the first contact portion body 411 may be formed of a resin material including a specific additive. The specific additive may be a material that is nonconductive and then turns conductive when light of a specific condition is radiated thereto. Then, light (a laser) of a specific condition may be radiated to a region on a surface of the first contact portion body 411 where the first signal transfer portion 415 is to be formed. Accordingly, the additive in the region to which the light has been radiated may turn conductive. Then, when the first contact portion body 411 is immersed in a plating solution, a plating layer is selectively formed only on the region on the surface of the first contact portion body 411 to which the light has been radiated.

In another example, the first signal transfer portions 415 may be plating layers formed using a selective etching method. Specifically, the first contact portion 410 may be an injection molded part consisting of a first resin material and a second resin material having different chemical characteristics. The first contact portion 410 made of two different kinds of resin materials may be injected molded through a double injection molding method. Here, a region on a surface of the first contact portion body 411 where the first signal transfer portion 415 is to be formed may be formed of the first resin material. When the first contact portion 410 is immersed in a specific etching solution, etching is performed only on the region on the surface that is formed of the first resin material reacting to the specific etching solution. Then, when the selectively-etched first contact portion body 411 is immersed in a plating solution, a plating layer is selectively formed only on the etched region on the surface of the first contact portion body 411.

The first signal transfer portions 415 may be formed as plating layers coupled to a surface of the first contact portion body 411 using various methods other than those described above.

The second contact portion 420 may be formed of a structure identical or similar to the structure of the first contact portion 410. The second contact portion 420 includes a second contact portion body 421 and a second signal transfer portion 425. The second contact portion body 421 may include a second contact surface 422, a second circuit connecting surface 423, and a second coupling surface 424. The description of the structure of the second contact portion 420 will be substituted by the description of the first contact portion 410.

The contact portion 401 may include the fastening portions 412a and 422a. The fastening portions 412a and 422a may be configurations of portions where the first contact portion 410 and the second contact portion 420 are coupled. Specifically, the first contact portion 410 and the second contact portion 420 may respectively include the fastening portions 412a and 422a fitted and coupled to each other. The fastening portions 412a and 422a may be formed at portions of the first contact portion 410 and the second contact portion 420 that come into contact with each other.

Specifically, the fastening portions 412a and 422a may be formed at the first contact surface 412 and the second contact surface 422. As illustrated in FIG. 27, a first fastening portion 412a in the form of a protrusion may be formed on the first contact surface 412, and a second fastening portion 422a in the form of a groove may be formed in the second contact surface 422. When the first contact portion 410 and the second contact portion 420 are coupled, the first and second fastening portions 412a and 422a may be fitted and coupled.

In addition, fastening portions 412a' and 422a' may also be formed at the first coupling surface 414 and the second coupling surface 424. As illustrated in FIG. 27, a first fastening portion 412a' in the form of a groove may be formed in the first coupling surface 414, and a second fastening portion 422a' in the form of a protrusion may be formed on the second coupling surface 424. When the first contact portion 410 and the second contact portion 420 are coupled, the first and second fastening portions 412a' and 422a' may be fitted and coupled.

FIG. 28 is an exploded perspective view of the first contact portion, the second contact portion, and the sensor portion according to one additional embodiment of the present invention, and FIG. 29 is a perspective view of a state in which the first contact portion, the second contact portion, and the sensor portion are coupled according to one additional embodiment of the present invention.

Referring to FIGS. 28 and 29, the first contact portion 410 and the second contact portion 420 may be in close contact with each other with the connecting portion 210 disposed therebetween and may be coupled. Accordingly, the first surface 211 of the connecting portion 210 comes into contact with the first contact surface 412 of the first contact portion 410, and the second surface 212 of the connecting portion 210 comes into contact with the second contact surface 422 of the second contact portion 420.

The connecting portion 210 may be formed in the form of a plate that stands upright in the vertical direction on the lower surface of the housing 100. In addition, the first contact surface 412 and the second contact surface 422 may also be formed as vertical surfaces that stand upright in the vertical direction on the lower surface. Therefore, the first contact surface 412, the connecting portion 210, and the second contact surface 422 may come into close contact with one another in the horizontal direction.

The first signal transfer portion 415 may extend from the sensor connecting terminal 415a to the circuit connecting terminal 415b. The sensor connecting terminal 415a of the first signal transfer portion 415 may be formed on the first contact surface 412. In addition, the circuit connecting terminal 415b may be formed on the first circuit connecting surface 413.

The sensor connecting terminal 415a of the first signal transfer portion 415 may be in contact with the first sensor terminals 231 and 232 formed on the first surface 211 of the connecting portion 210, and the circuit connecting terminal 415b may be connected to the signal terminal 310 of the circuit portion 300. In this way, the first signal transfer portion 415 may electrically connect the first sensor terminals 231 and 232 and the circuit portion 300.

The second signal transfer portion 425 may be formed in the same manner as the first signal transfer portion 415. A sensor connecting terminal 425a of the second signal transfer portion 425 may be in contact with the second sensor terminal 233 formed on the second surface 212 of the connecting portion 210, and a circuit connecting terminal 425b may be connected to the signal terminal 310 of the circuit portion 300. In this way, the second signal transfer portion 425 may electrically connect the second sensor terminal 233 and the circuit portion 300.

The connecting portion 210 of the sensor portion 200 may include the sensor fastening portion 215. The sensor fastening portion 215 may be coupled to the fastening portions 412a and 422a. The sensor fastening portion 215 may be formed in the form of a groove or an opening. When the first contact portion 410 and the second contact portion 420 are coupled, the fastening portions 412a and 422a may be fitted and coupled. When the fastening portions 412a and 422a are coupled, the first fastening portion 412a formed with a protruding structure may be coupled by passing through the sensor fastening portion 215. Due to the sensor fastening portion 215 and the fastening portions 412a and 422a being coupled, the position of the connecting portion 210 may be fixed between the first contact portion 410 and the second contact portion 420.

Referring to FIG. 29, when the first contact portion 410 and the second contact portion 420 are coupled, a sensor accommodating space 402 may be formed in the contact portion 401. The connecting portion 210 may extend from between the first contact surface 412 and the second contact surface 422 to the sensor accommodating space 402 and may extend to the sensing portion 220.

When the first contact portion 410 and the second contact portion 420 are coupled, the first circuit connecting surface 413 and the second circuit connecting surface 423 may be located to be coplanar. Therefore, the circuit connecting terminals 415b and 425b formed on the first circuit connecting surface 413 and the second circuit connecting surface 423 may be coupled by solder etc., to a plurality of signal terminals 310 arranged in a row.

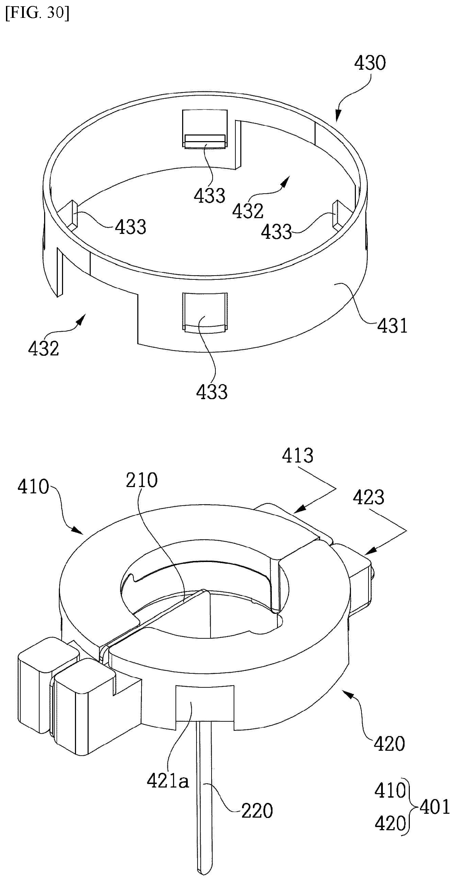

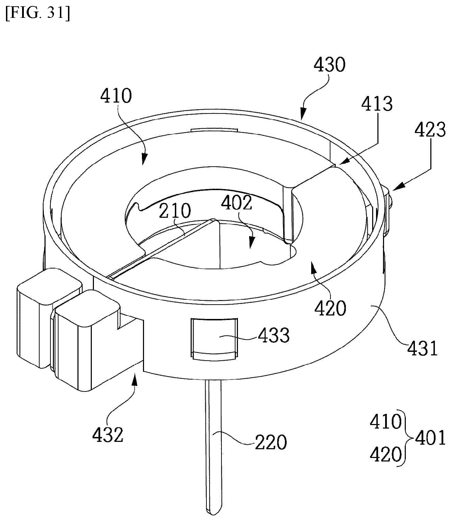

FIG. 30 is a partial exploded perspective view of the sensor portion, the contact portion, and the close contact maintaining portion according to one additional embodiment of the present invention, and FIG. 31 is a perspective view of a state in which the sensor portion, the contact portion, and the close contact maintaining portion are coupled according to one additional embodiment of the present invention.

Referring to FIG. 30, the sensor holder 400 may include the close contact maintaining portion 430. The close contact maintaining portion 430 may be coupled to the contact portion 401. Specifically, the close contact maintaining portion 430 may maintain the close contact state between the first contact portion 410 and the second contact portion 420. The close contact maintaining portion 430 may allow the first contact portion 410 and the second contact portion 420 to come into close contact in directions facing each other and may further strengthen the close contact among the first contact surface 412, the connecting portion 210, and the second contact surface 422 in the horizontal direction.

The close contact maintaining portion 430 may include a bracket 431 and an elastic portion 433.

The bracket 431 may be formed in the form of a ring that surrounds at least a portion of the contact portion 401. When the close contact maintaining portion 430 is coupled to the contact portion 401, the bracket 431 may be located to surround at least a portion of the contact portion 401 from outside the contact portion 401.

The elastic portion 433 may be located inside the bracket 431. The elastic portion 433 may extend inward from the bracket 431 and may be formed to have elasticity. The elastic portion 433 may be coupled to an elastic portion fastening portion 421a of the contact portion 401.

As illustrated in FIG. 31, when the close contact maintaining portion 430 is coupled to the contact portion 401, the elastic portion 433 may be deformed. The elastic portion 433 may, due to a restoring force, apply an external force pushing the contact portion 401 toward the inside of the bracket 431. Accordingly, the first contact portion 410 and the second contact portion 420 may come into close contact in directions facing each other.

At least one open portion 432 may be formed in the bracket 431 of the close contact maintaining portion 430. The open portion 432 may be formed as an opening that connects the inside and the outside of the bracket. When the close contact maintaining portion 430 is coupled to the contact portion 401, at least a portion of the contact portion 401 may extend from the inside of the bracket 431 to the outside through the open portion 432.

As illustrated in FIG. 31, portions of the circuit connecting surfaces 413 and 423 of the contact portion 401 may extend to the outside of the bracket 431 through the open portion 432. In this way, the circuit connecting surfaces 413 and 423 may be located outside the bracket 431 and be connected to the circuit portion 300.

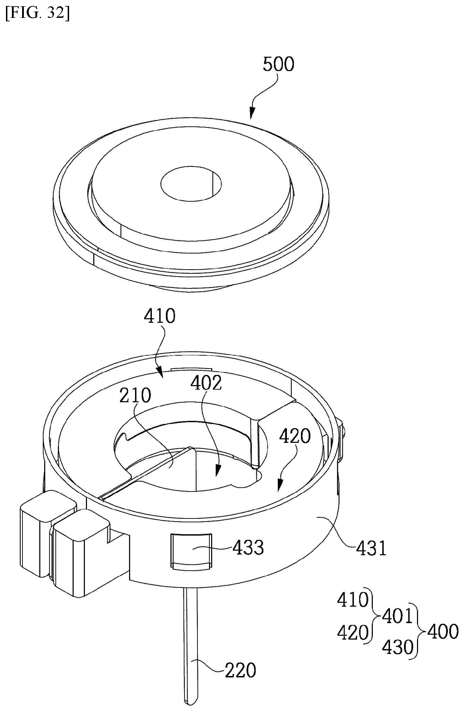

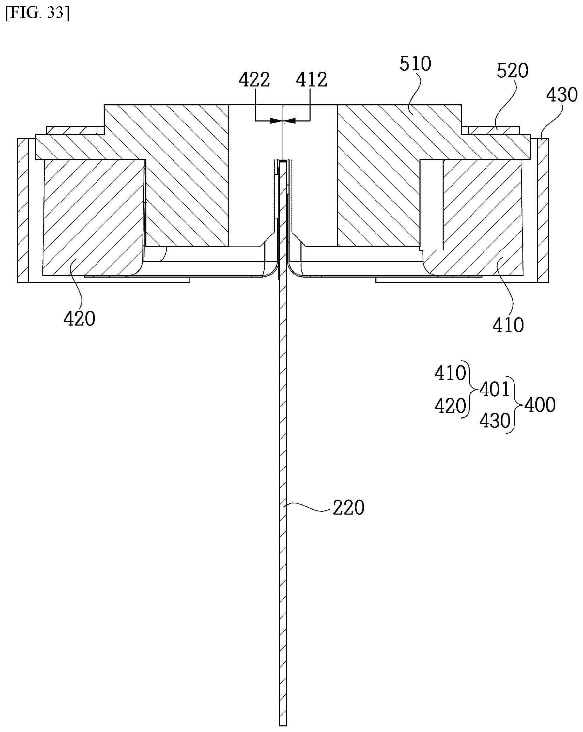

FIG. 32 is a partial exploded perspective view of the sensor portion, the sensor holder, and the sensor fixing portion according to one additional embodiment of the present invention, and FIG. 33 is a cross-sectional view of a state in which the sensor portion, the sensor holder, and the sensor fixing portion are coupled according to one additional embodiment of the present invention.

Referring to FIG. 32, when the sensor portion 200 is coupled to the sensor holder 400, the sensor accommodating space 402 is formed inside the sensor holder 400. The sensor portion 200 may extend in the sensor accommodating space 402.

Referring to FIGS. 32 and 33, the sensor fixing portion 500 may be inserted into the sensor accommodating space 402. The sensor fixing portion 500 may be coupled to the sensor portion 200 in the sensor accommodating space 402. The position of the sensor portion 200 may be fixed due to the sensor fixing portion 500.

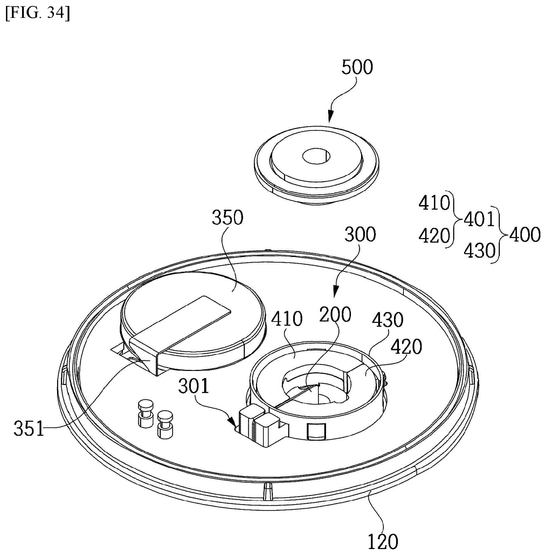

FIG. 34 is an exploded perspective view of a state in which the upper housing is open in the analyte monitoring device according to one additional embodiment of the present invention.

Referring to FIG. 34, a coupling product of the sensor portion 200 and the sensor holder 400 may be coupled to the lower housing 120. As the coupling product of the sensor portion 200 and the sensor holder 400 is coupled to the lower housing 120, the sensing portion 220 of the sensor portion 200 may be exposed to the outside through the through-hole 121.

In addition, the circuit portion 300 may be coupled to the lower housing 120 so that the coupling product of the sensor portion 200 and the sensor holder 400 is accommodated in the opening 301. The signal terminal 310 of the circuit portion 300 may be located to be adjacent to the circuit connecting terminals 415b and 425b. The signal terminal 310 of the circuit portion 300 may be electrically connected by solder etc., to the circuit connecting terminals 415b and 425b.

The sensor fixing portion 500 may be inserted into the sensor accommodating space 402. The sensor fixing portion 500 may be coupled to the sensor portion 200 in the sensor accommodating space 402. The position of the sensor portion 200 may be fixed due to the sensor fixing portion 500.

In FIG. 33, the sensor portion 200, the sensor holder 400, and the sensor fixing portion 500 being coupled first and a coupling product thereof being coupled to the lower housing 120 is illustrated. However, in FIG. 34, the sensor portion 200 and the sensor holder 400 being coupled first, the coupling product thereof being coupled to the lower housing 120, and then the sensor fixing portion 500 being coupled is illustrated. In the present invention, an order of coupling of each configuration is not limited.

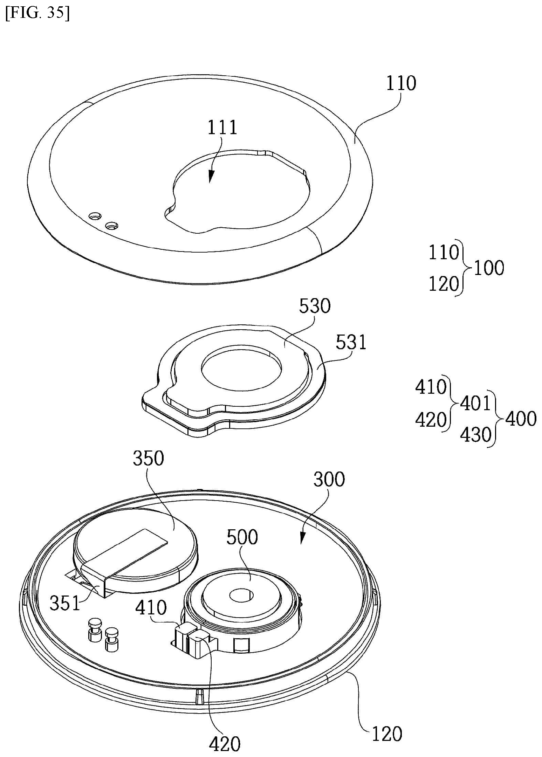

FIG. 35 is an exploded perspective view of the analyte monitoring device according to one additional embodiment of the present invention.

Referring to FIG. 35, the sensor portion 200, the sensor holder 400, and the sensor fixing portion 500 may be coupled to the lower housing 120. In addition, the circuit portion 300 may also be coupled to the lower housing 120, and the signal terminal 310 and the circuit connecting terminals 415b and 425b may be electrically connected. In this way, the circuit portion 300 may obtain sensing data of the sensor portion 200. Then, a fixing portion cover 530 and the upper housing 110 may be additionally coupled.

The fixing portion cover 530 may be coupled to an upper portion of the sensor holder 400 and the sensor fixing portion 500. In addition, the upper housing 110 may be coupled to the lower housing 120. The upper opening 111 may be formed in the upper housing 110. The upper housing 111 may be coupled to the fixing portion cover 530. An edge portion of the fixing portion cover 530 may be located at a lower portion of a circumferential surface of the upper opening 111 and may be coupled by a waterproof tape 531 or the like. Accordingly, the inner space of the housing 100 may remain waterproof from the outside.

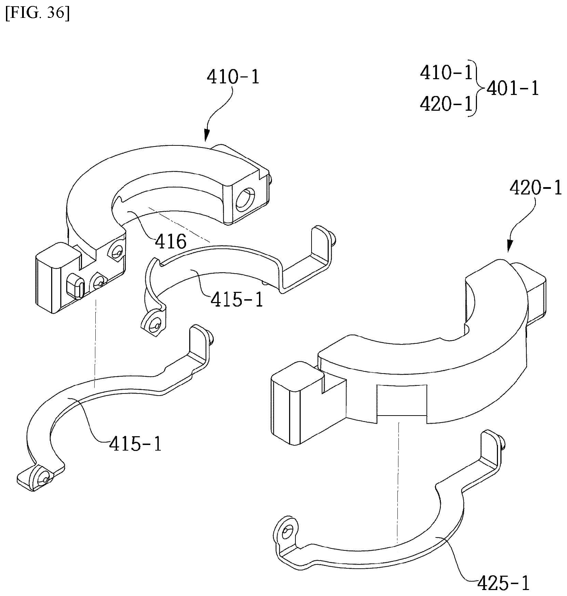

Hereinafter, an analyte monitoring device according to another embodiment of the present invention will be described with reference to FIG. 36.

FIG. 36 is an exploded perspective view of a contact portion according to another embodiment of the present invention. A contact portion 401-1 illustrated in FIG. 36 will be described focusing on differences from the contact portion 401 illustrated in FIG. 27.

Referring to FIG. 36, the contact portion 401-1 may include a first contact portion 410-1 and a second contact portion 420-1 separated from each other. In FIG. 36, the first contact portion 410-1 and the second contact portion 420-1 both serving as transfer contact portions is illustrated.

A first signal transfer portion 415-1 may be coupled to a first contact portion body 411-1. The first signal transfer portion 415-1 is formed of a conductive material. Specifically, the first signal transfer portion 415-1 may be formed as a metal plate processed to be bent in a specific shape. The first signal transfer portion 415-1 may be a metal plate press-processed to be coupled to a surface of the first contact portion body 411-1. A mounting portion 416 to which the first signal transfer portion 415-1 can be coupled may be formed on the surface of the first contact portion body 411-1. The mounting portion 416 may be formed in the form of a groove that is recessed at a depth corresponding to a thickness of the first signal transfer portion 415-1. When the first signal transfer portion 415-1 is formed as a metal plate, the first signal transfer portion 415-1 can be formed with a thicker thickness compared to when being formed as a plating layer.

The second contact portion 420-1 may be formed of a structure identical or similar to the structure of the first contact portion 410-1. Specifically, a second signal transfer portion 425-1 may also be formed as a metal plate processed to be bent in a specific shape. The second signal transfer portion 425-1 may be coupled to a surface of a second contact portion body 421-1.

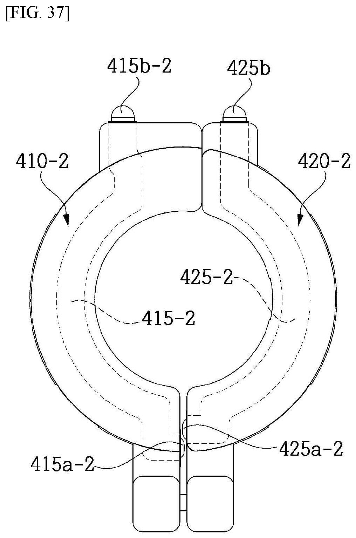

Hereinafter, an analyte monitoring device according to still another embodiment of the present invention will be described with reference to FIG. 37.

FIG. 37 is a see-through top view of a contact portion according to still another embodiment of the present invention. A contact portion 401-2 illustrated in FIG. 37 will be described focusing on differences from the contact portion 401 illustrated in FIG. 27.

A first signal transfer portion 415-2 may be coupled to a first contact portion body 411-2 with at least a portion of the first signal transfer portion 415-2 being buried in the first contact portion body 411-2. Referring to FIG. 37, while portions of a sensor connecting terminal 415a-2 and a circuit connecting terminal 415b-2 of the first signal transfer portion 415-2 are exposed to a surface of the first contact portion body 411-2, other portions may be located to be buried in the first contact portion body 411-2.

The first contact portion body 411-2 may be formed to be injection molded (insert molded) to allow the first signal transfer portion 415-2 to be inserted thereinto. In addition, in some cases, the first signal transfer portion 415-2 may be coupled by being inserted into the first contact portion body 411-2.

A second contact portion 420-2 and a second signal transfer portion 425-2 may be formed of structures identical or similar to the structures of a first contact portion 410-2 and the first signal transfer portion 415-2. Specifically, the second signal transfer portion 425-2 may be coupled to a second contact portion body 421-2 with at least a portion of the second signal transfer portion 425-2 being buried in the second contact portion body 421-2. While portions of a sensor connecting terminal 425a-2 and a circuit connecting terminal 425b-2 of the second signal transfer portion 425-2 are exposed to a surface of the second contact portion body 421-2, other portions may be located to be buried in the second contact portion body 421-2.

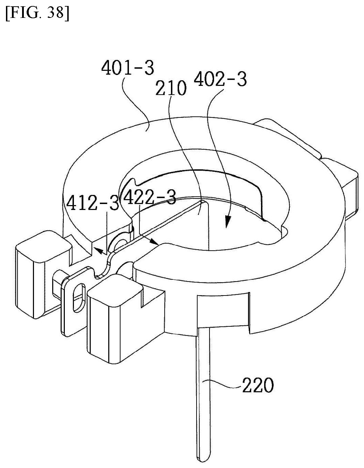

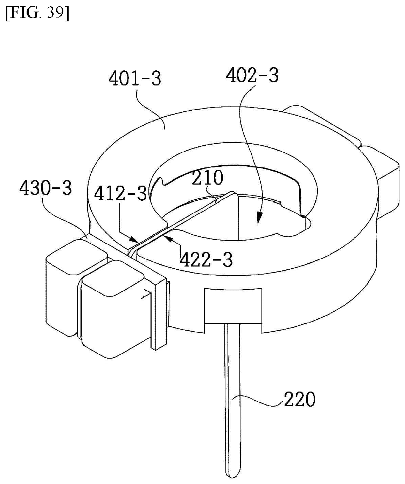

Hereinafter, an analyte monitoring device according to yet another embodiment of the present invention will be described with reference to FIGS. 38 and 39.

FIG. 38 is an exploded perspective view of a sensor portion and a contact portion according to yet another embodiment of the present invention, and FIG. 39 is a perspective view of a coupling state of the sensor portion and a sensor holder according to yet another embodiment of the present invention.

Referring to FIG. 38, a contact portion 401-3 may be a single configuration formed as one body. The contact portion 401-3 may have a slit (a gap) formed at one side. The contact portion 401-3 may be a C-shaped configuration having a sensor accommodating space 402-3 formed therein and having an open slit portion.

In the contact portion 401-3, a first contact portion 412-3 and a second contact portion 422-3 may be formed as contact surfaces that come into contact with both surfaces of the connecting portion 210. A portion between the first contact portion 412-3 and the second contact portion 422-3 may correspond to the slit.

Referring to FIG. 39, in the contact portion 401-3, the slit may be closed as the first contact portion 412-3 and the second contact portion 422-3 come into close contact. The connecting portion 210 may be located in the slit, and both surfaces of the connecting portion 210 may be in close contact with and coupled to the first contact portion 412-3 and the second contact portion 422-3. As the slit is closed, the sensor accommodating space 402-3 in the contact portion 401-3 may become a space that is closed in the horizontal direction. The connecting portion 210 may extend in the sensor accommodating space 402-3 and may extend to the sensing portion 220.

A close contact maintaining portion 430-3 may be formed as an anti-opening clip of the slit and may be coupled in the vicinity of the first contact portion 412-3 and the second contact portion 422-3 to allow the first contact portion 412-3 and the second contact portion 422-3 to come into close contact with each other.

In an analyte monitoring device having the above-described configuration, there are advantages in that a sensor portion can be stably coupled by a sensor holder, and the sensor portion and a circuit portion can be electrically connected using a simple method. In addition, there is an advantage in that configurations for electrical connection between the sensor holder and the sensor portion and between the sensor holder and the circuit portion are simple and sturdy, thus having excellent durability.

The technical features disclosed in each embodiment of the present invention are not limited only to the corresponding embodiment, and the technical features disclosed in each embodiment may be combined and applied to different embodiments unless they are mutually incompatible.

Therefore, although description of each embodiment focuses on technical features of each embodiment, the technical features may be combined and applied unless they are mutually incompatible.

The present invention is not limited to the above-described embodiments and the accompanying drawings, and various modifications and variations can be made by those of ordinary skill in the art to which the present invention pertains. Therefore, the scope of the present invention should be defined not only by the claims of this specification but also by their equivalents.

## Claims

1. An analyte monitoring device comprising:
a housing forming an inner space;
a sensor portion including a connecting portion located in the inner space and a sensing portion extending from the connecting portion and exposed to the outside of the housing; and
a circuit portion located in the inner space and including a contact portion in contact with the connecting portion,
wherein the contact portion is formed of a flexible material and is formed to extend in a horizontal direction from a portion adjacent to a body portion of the circuit portion and then bend upward or downward due to contact with the connecting portion.

2. The analyte monitoring device of claim 1, wherein the body portion is formed as a rigid circuit board.

3. The analyte monitoring device of claim 2, wherein:
the body portion is formed as a circuit board including a plurality of layers that are stacked; and
the contact portion is formed as a flexible conductive member that extends from at least one of the plurality of layers.

4. The analyte monitoring device of claim 2, wherein the contact portion is a flexible circuit board coupled to the body portion.

5. The analyte monitoring device of claim 1, wherein the contact portion includes:
a horizontal extending portion that is adjacent to the body portion and extends in the horizontal direction; and
a bending portion that extends from an end of the horizontal extending portion and bends upward or downward due to the contact with the connecting portion.

6. The analyte monitoring device of claim 1, wherein the contact portion further includes a contact extending portion that extends from an end of the bending portion and extends parallel to the connecting portion while in contact therewith.

7. The analyte monitoring device of claim 1, wherein the connecting portion is formed in the form of a plate that stands upright in a vertical direction.

8. The analyte monitoring device of claim 7, wherein:
the connecting portion includes a sensor terminal formed on a surface facing the contact portion;
the contact portion includes a contact terminal formed on a lower surface; and
the sensor terminal and the contact terminal come into contact as the contact portion bends upward due to the contact with the connecting portion.

9. The analyte monitoring device of claim 7, wherein:
the connecting portion includes a first sensor terminal formed on a first surface and a second sensor terminal formed on a second surface opposite to the first surface; and
the contact portion includes a first contact portion that extends from a first end of the body portion toward the first surface and comes into contact with the first sensor terminal and a second contact portion that extends from a second end of the body portion toward the second surface and comes into contact with the second sensor terminal.

10. The analyte monitoring device of claim 1, further comprising a contact maintaining portion that maintains a contact state between the connecting portion and the contact portion.

11. The analyte monitoring device of claim 10, wherein the contact maintaining portion includes:
an extending portion that extends in the inner space of the housing; and
a coupling portion that is coupled to the extending portion so that the connecting portion and the contact portion that are in contact with each other are located between the coupling portion and the extending portion.

12. The analyte monitoring device of claim 11, wherein:
the extending portion includes a protruding portion that protrudes from the housing and comes into contact with any one of the connecting portion and the contact portion and a spaced portion that protrudes from the housing and is spaced from the protruding portion; and
the coupling portion is coupled between the protruding portion and the spaced portion, comes into contact with the other of the connecting portion and the contact portion, and allows the connecting portion and the contact portion to come in close contact with each other.

13. The analyte monitoring device of claim 11, wherein:
the extending portion includes a protruding portion that protrudes from the housing and comes into contact with any one of the connecting portion and the contact portion; and
the coupling portion rotates about a rotating shaft to be adjacent to the protruding portion, comes into contact with the other of the connecting portion and the contact portion, and allows the connecting portion and the contact portion to come in close contact with each other.

14. The analyte monitoring device of claim 11, wherein:
the connecting portion includes a first sensor terminal formed on a first surface of a first portion of the connecting portion and a second sensor terminal formed on a second surface of a second portion of the connecting portion, the first surface and the second surface being surfaces opposite to each other;
the contact portion includes a first contact portion that extends from a first end of the body portion toward the first surface and comes into contact with the first sensor terminal and a second contact portion that extends from a second end of the body portion toward the second surface and comes into contact with the second sensor terminal;
the extending portion includes a first protruding portion that protrudes from the housing and comes into contact with a second surface of the first portion and a second protruding portion that protrudes from the housing and comes into contact with a first surface of the second portion; and
the coupling portion includes a first coupling portion that is coupled to the second protruding portion in a direction toward the first surface and maintains a contact state between the first sensor terminal and the first contact portion and a second coupling portion that is coupled to the first protruding portion in a direction toward the second surface and maintains a contact state between the second sensor terminal and the second contact portion.

15. The analyte monitoring device of claim 11, further comprising a coupling maintaining portion that maintains a coupling state between the extending portion and the coupling portion.

16. An analyte monitoring device comprising:
a housing forming an inner space;
a sensor portion including a connecting portion located in the inner space and having at least one sensor terminal formed thereon and a sensing portion extending from the connecting portion and exposed to the outside of the housing;
a circuit portion located in the inner space and including a signal processing portion; and
a sensor holder that includes a first contact portion and a second contact portion in contact with a first surface and a second surface, respectively, of the connecting portion and electrically connects the sensor terminal and the circuit portion.

17. The analyte monitoring device of claim 16, wherein at least one of the first contact portion and the second contact portion is a transfer contact portion including a signal transfer portion connected to the sensor terminal and the circuit portion.

18. The analyte monitoring device of claim 17, wherein:
the transfer contact portion is formed of a resin material including an additive that is nonconductive and then turns conductive when light of a specific condition is radiated thereto;
the transfer contact portion includes, on one portion of a surface thereof, a light radiation region in which the additive turns conductive due to the light of the specific condition being radiated thereto; and
the transfer contact portion is a plated layer coupled to the light radiation region.

19. The analyte monitoring device of claim 16, wherein the sensor holder further includes a close contact maintaining portion that maintains a state in which the first contact portion and the second contact portion are in close contact with each other with the connecting portion disposed therebetween.

20. The analyte monitoring device of claim 16, wherein:
the sensor holder forms a sensor accommodating space accommodating a portion of the connecting portion that is adjacent to the sensing portion; and
a through-hole through which the sensing portion passes is formed in a portion of the housing that is located at a lower portion of the sensor accommodating space.
